# EUROPEAN PATENT APPLICATION

(11) **EP 4 357 341 A1**
(43) Date of publication of application: **24.04.2024**
(21) Application number: 22911695.9
(22) Date of filing: 07.12.2022
(51) Int. Cl.: C07D 405/04, C07D 405/14, C07D 409/14, C07D 409/04, C07D 495/04, H10K 99/00, H10K 50/00, C09K 11/06

(54) **NOVEL COMPOUND AND ORGANIC LIGHT EMITTING DEVICE COMPRISING SAME**

(30) Priority: 22.12.2021 KR 20210185368
(71) Applicant: LG Chem, Ltd., Yeongdeungpo-gu Seoul 07336 (KR)
(72) Inventor: KIM, Hoon Jun, Daejeon 34122 (KR); LEE, Dong Hoon, Daejeon 34122 (KR); HAN, Miyeon, Daejeon 34122 (KR); JUNG, Min Woo, Daejeon 34122 (KR); PARK, Seulchan, Daejeon 34122 (KR); CHO, Hye Min, Daejeon 34122 (KR); LEE, Hojung, Daejeon 34122 (KR)
(74) Representative: Hoffmann Eitle
(86) International application number: PCT/KR2022/019784
(87) International publication number: WO 2023/121062

(57) **Abstract**

The present disclosure relates to a novel compound and an organic light emitting device including the same.

## Description

### [TECHNICAL FIELD]

### Cross-reference to Related Application(s)

This application claims the benefit of Korean Patent Application No. 10-2021-0185368 filed on December 22, 2021 in the Korean Intellectual Property Office, the disclosures of which are incorporated herein by reference in their entirety.

The present disclosure relates to a novel compound and an organic light emitting device including the same.

### [BACKGROUND OF ART]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer, and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

There is a continuing need for the development of new materials for the organic materials used in the organic light emitting devices as described above.

### [PRIOR ART LITERATURE]

(Patent Literature 0001) Korean Unexamined Patent Publication No. 10-2000-0051826

### [DETAILED DESCRIPTION OF THE INVENTION]

### [Technical Problem]

The present disclosure relates to a novel compound and an organic light emitting device including the same.

### [Technical Solution]

In the present disclosure, there is provided a compound represented by the following Chemical Formula 1: 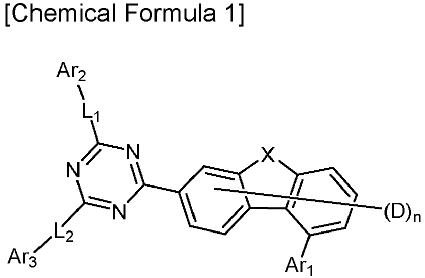 in the Chemical Formula 1,
n is an integer of 1 to 6,
X is O or S,
L₁ and L₂ are each independently a single bond; substituted or unsubstituted C₆₋₆₀ arylene; or substituted or unsubstituted C₂₋₆₀ heteroarylene containing at least one selected from the group consisting of O, S, Si, P and B,
Ar₁ is cyano; substituted or unsubstituted C₆₋₆₀ aryl; or substituted or unsubstituted C₂₋₆₀ heteroaryl containing at least one selected from the group consisting of O, S, Si, P and B, and
Ar₂ and Ar₃ are each independently substituted or unsubstituted C₆₋₆₀ aryl; or substituted or unsubstituted C₂₋₆₀ heteroaryl containing at least one selected from the group consisting of O, S, Si, P and B.

In addition, there is provided an organic light emitting device including: a first electrode; a second electrode that is provided opposite to the first electrode; and one or more organic material layers that are provided between the first electrode and the second electrode, wherein at least one layer of the organic material layers includes the compound represented by the Chemical Formula 1.

### [ADVANTAGEOUS EFFECTS]

The compound represented by the Chemical Formula 1 may be used as a material for an organic material layer of an organic light emitting device, and may improve efficiency, low driving voltage, and/or lifespan of the organic light emitting device. In particular, the compound represented by the Chemical Formula 1 may be used as a material for hole injection, hole transport, hole injection and transport, electron blocking, light emission, electron transport, or electron injection.

### [BRIEF DESCRIPTION OF THE DRAWINGS]

FIG. 1 shows an example of an organic light emitting device including a substrate 1, an anode 2, an organic material layer 3, and a cathode 4.
FIG. 2 shows an example of an organic light emitting device including a substrate 1, an anode 2, a hole injection layer 5, a hole transport layer 6, an electron blocking layer 7, a light emitting layer 8, a hole blocking layer 9, an electron transport layer 10, an electron injection layer 11, and a cathode 4.
FIG. 3 shows an example of an organic light emitting device including a substrate 1, an anode 2, a hole injection layer 5, a hole transport layer 6, an electron blocking layer 7, a light emitting layer 8, a hole blocking layer 9, an electron injection and transport layer 12, and a cathode 4.

### [DETAILED DESCRIPTION OF THE EMBODIMENTS]

Hereinafter, embodiments of the present disclosure will be described in more detail to facilitate understanding of the invention.

In the present disclosure, a compound represented by the Chemical Formula 1 above is provided.

As used herein, the notation 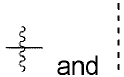 means a bond linked to another substituent group.

As used herein, the term "substituted or unsubstituted" means being unsubstituted or substituted with one or more substituents selected from the group consisting of deuterium; a halogen group; a nitrile group; a nitro group; a hydroxyl group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heterocyclic group containing at least one of N, O and S atoms, or being unsubstituted or substituted with a substituent in which two or more substituents of the above-exemplified substituents are connected. For example, "a substituent in which two or more substituents are connected" may be a biphenyl group. Namely, a biphenyl group may be an aryl group, or it may also be interpreted as a substituent in which two phenyl groups are connected.

In the present disclosure, the carbon number of a carbonyl group is not particularly limited, but is preferably 1 to 40. Specifically, the carbonyl group may be a compound having the following structural formulae, but is not limited thereto.

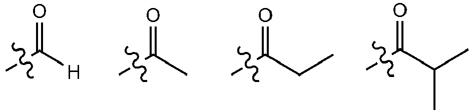

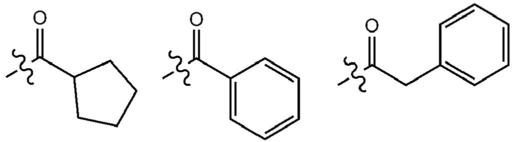

In the present disclosure, an ester group may have a structure in which oxygen of the ester group is substituted by a straight-chain, branched-chain, or cyclic alkyl group having 1 to 25 carbon atoms, or an aryl group having 6 to 25 carbon atoms. Specifically, the ester group may be a compound having the following structural formulae, but is not limited thereto.

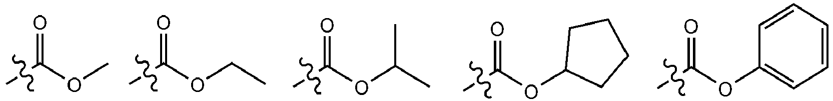

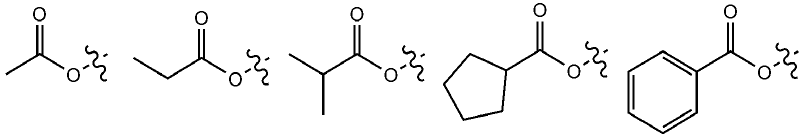

In the present disclosure, the carbon number of an imide group is not particularly limited, but is preferably 1 to 25. Specifically, the imide group may be a compound having the following structural formulae, but is not limited thereto.

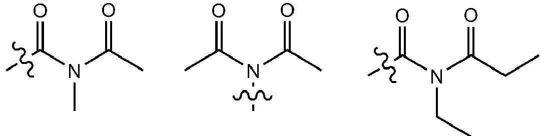

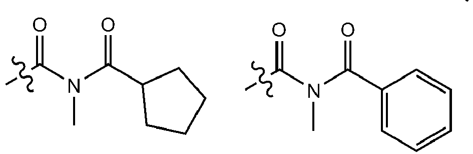

In the present disclosure, a silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but is not limited thereto.

In the present disclosure, a boron group specifically includes a trimethylboron group, a triethylboron group, a t-butyldimethylboron group, a triphenylboron group, a phenylboron group, and the like, but is not limited thereto.

In the present disclosure, examples of a halogen group include fluorine, chlorine, bromine, or iodine.

In the present disclosure, the alkyl group may be straight-chain, or branched-chain, and the carbon number thereof is not particularly limited, but is preferably 1 to 40. According to one embodiment, the carbon number of the alkyl group is 1 to 20. According to another embodiment, the carbon number of the alkyl group is 1 to 10. According to another embodiment, the carbon number of the alkyl group is 1 to 6. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethylpropyl, isohexyl, 2-methylpentyl, 4-methylhexyl, 5-methylhexyl, and the like, but are not limited thereto.

In the present disclosure, the alkenyl group may be straight-chain or branched-chain, and the carbon number thereof is not particularly limited, but is preferably 2 to 40. According to one embodiment, the carbon number of the alkenyl group is 2 to 20. According to another embodiment, the carbon number of the alkenyl group is 2 to 10. According to another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1 ,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present disclosure, a cycloalkyl group is not particularly limited, but the carbon number thereof is preferably 3 to 60. According to one embodiment, the carbon number of the cycloalkyl group is 3 to 30. According to another embodiment, the carbon number of the cycloalkyl group is 3 to 20. According to another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present disclosure, an aryl group is not particularly limited, but the carbon number thereof is preferably 6 to 60, and it may be a monocyclic aryl group or a polycyclic aryl group. According to one embodiment, the carbon number of the aryl group is 6 to 30. According to one embodiment, the carbon number of the aryl group is 6 to 20. The monocyclic aryl group includes a phenyl group, a biphenyl group, a terphenyl group and the like, but is not limited thereto. The polycyclic aryl group includes a naphthyl group, an anthracenyl group, a phenanthryl group, a pyrenyl group, a perylenyl group, a chrysenyl group, a fluorenyl group or the like, but is not limited thereto.

In the present disclosure, a fluorenyl group may be substituted, and two substituents may be bonded to each other to form a spiro structure. In the case where the fluorenyl group is substituted, 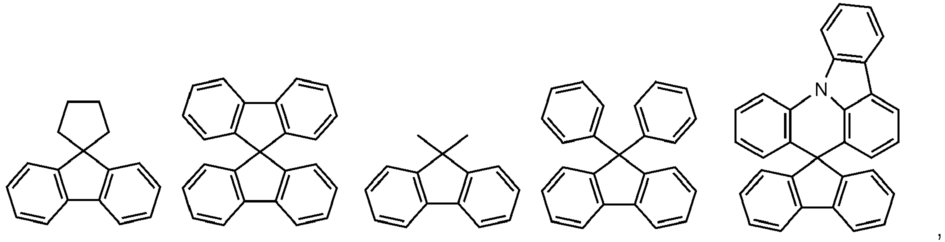 and the like can be formed. However, the structure is not limited thereto.

In the present disclosure, a heterocyclic group is a heterocyclic group containing at least one heteroatom of O, N, Si and S as a heterogeneous element, and the carbon number thereof is not particularly limited, but is preferably 2 to 60. Examples of the heterocyclic group include a thiophene group, a furan group, a pyrrole group, an imidazole group, a thiazole group, an oxazol group, an oxadiazol group, a triazol group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzoimidazole group, a benzothiazol group, a benzocarbazole group, a benzothiophene group, a dibenzothiophene group, a benzofuranyl group, a phenanthroline group, an isoxazolyl group, a thiadiazolyl group, a phenothiazinyl group, a dibenzofuranyl group, and the like, but are not limited thereto.

In the present disclosure, the aryl group in the aralkyl group, the aralkenyl group, the alkylaryl group, and the arylamine group is the same as the aforementioned examples of the aryl group. In the present disclosure, the alkyl group in the aralkyl group, the alkylaryl group and the alkylamine group is the same as the aforementioned examples of the alkyl group. In the present disclosure, the heteroaryl in the heteroarylamine can apply the aforementioned description of the heterocyclic group. In the present disclosure, the alkenyl group in the aralkenyl group is the same as the aforementioned examples of the alkenyl group. In the present disclosure, the aforementioned description of the aryl group may be applied except that the arylene is a divalent group. In the present disclosure, the aforementioned description of the heterocyclic group can be applied except that the heteroarylene is a divalent group. In the present disclosure, the aforementioned description of the aryl group or cycloalkyl group can be applied except that the hydrocarbon ring is not a monovalent group but formed by combining two substituent groups. In the present disclosure, the aforementioned description of the heterocyclic group can be applied, except that the heterocycle is not a monovalent group but formed by combining two substituent groups.

The compound represented by the Chemical Formula 1 includes dibenzofuran or dibenzothiophene containing at least one deuterium, and an aryl- or heteroaryl-substituted triazine group bonded thereto; and an aryl or heteroaryl group, and does not include a nitrogen-containing heterocyclic group other than the triazine group. A compound satisfying the structure of Chemical Formula 1 exhibits low voltage when applied to an organic light emitting device and has excellent efficiency and lifespan.

The Chemical Formula 1 may be specifically represented by the following Chemical Formula 1-1 or 1-2: 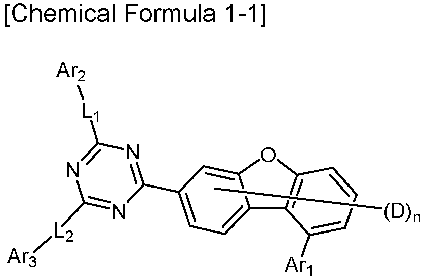 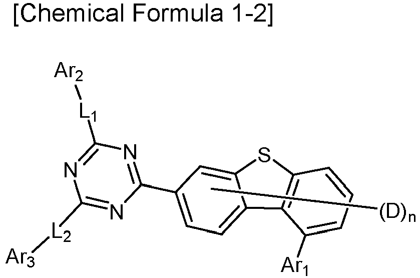 in the Chemical Formulae 1-1 and 1-2,
n, L₁, L₂, and Ar₁ to Ar₃ are as defined in the Chemical Formula 1.

Preferably, n is an integer of 4 to 6.

In the Chemical Formula 1, at least one hydrogen of L₁, L₂, and Ar₁ to Ar₃ may be substituted with deuterium.

Preferably, L₁ and L₂ are each independently a single bond; substituted or unsubstituted C₆₋₂₀ arylene; or substituted or unsubstituted C₂₋₂₀ heteroarylene containing at least one selected from the group consisting of O, S, Si, P and B. Preferably, the heteroatom of the heteroaryl may be O and/or S.

Preferably, L₁ and L₂ are each independently a single bond; phenylene; or biphenylylene, and L₁ and L₂ may each independently be unsubstituted or substituted with at least one deuterium.

Preferably, L₁ and L₂ are each independently a single bond; phenylene unsubstituted or substituted with at least one deuterium; or biphenylylene unsubstituted or substituted with at least one deuterium.

Preferably, L₁ and L₂ are each independently a single bond; phenylene; or biphenylylene.

Preferably, Ar₁ is cyano; substituted or unsubstituted C₆₋₂₀ aryl; or substituted or unsubstituted C₂₋₂₀ heteroaryl containing at least one heteroatom selected from the group consisting of O, S, Si, P and B. Preferably, the heteroatom of the heteroaryl may be O and/or S. When Ar₁ is aryl or heteroaryl, at least one hydrogen may be substituted with deuterium.

Preferably, Ar₁ is cyano; phenyl; biphenylyl; terphenylyl; triphenylenyl; benzofuranyl; benzothiophenyl; dibenzofuranyl; or dibenzothiophenyl, and the substituents may be unsubstituted or substituted with at least one deuterium.

Preferably, Ar₁ is cyano; phenyl unsubstituted or substituted with at least one deuterium; biphenylyl unsubstituted or substituted with at least one deuterium; terphenylyl unsubstituted or substituted with at least one deuterium; triphenylenyl unsubstituted or substituted with at least one deuterium; benzofuranyl unsubstituted or substituted with at least one deuterium; benzothiophenyl unsubstituted or substituted with at least one deuterium; dibenzofuranyl unsubstituted or substituted with at least one deuterium; or dibenzothiophenyl unsubstituted or substituted with at least one deuterium.

Preferably, Ar₂ and Ar₃ are substituted or unsubstituted C₆₋₂₀ aryl; or substituted or unsubstituted C₂₋₂₀ heteroaryl containing at least one heteroatom selected from the group consisting of O, S, Si, P and B. Preferably, the heteroatom of the heteroaryl may be O and/or S. The Ar₂ and Ar₃ may each independently be substituted with at least one deuterium.

Preferably, Ar₂ and Ar₃ are each independently phenyl; phenyl substituted with at least one substituent selected from the group consisting of halogen, cyano, trimethylsilyl, C₁₋₄ alkyl, and C₁₋₄ alkenyl; biphenylyl; terphenylyl; naphthyl; 9,9-dimethylfluorenyl; 9,9-diphenylfluorenyl; triphenylenyl; chrysenyl; dibenzofuranyl; phenyldibenzofuranyl; dibenzothiophenyl; or phenyldibenzothiophenyl, and the substituents may be unsubstituted or substituted with at least one deuterium. Meanwhile, the 'phenyl substituted with at least one substituent selected from the group consisting of halogen, cyano, trimethylsilyl, C₁₋₄ alkyl, and C₁₋₄ alkenyl' may be, for example, fluorophenyl, cyanophenyl, trimethylsilanophenyl, dimethylphenyl, t-butylphenyl, or ethenylphenyl.

Preferably, Ar₂ and Ar₃ are each independently phenyl unsubstituted or substituted with at least one deuterium; fluorophenyl unsubstituted or substituted with at least one deuterium; cyanophenyl unsubstituted or substituted with at least one deuterium; trimethylsilanophenyl unsubstituted or substituted with at least one deuterium; dimethylphenyl unsubstituted or substituted with at least one deuterium; t-butylphenyl unsubstituted or substituted with at least one deuterium; ethenylphenyl unsubstituted or substituted with at least one deuterium; biphenylyl unsubstituted or substituted with at least one deuterium; terphenylyl unsubstituted or substituted with at least one deuterium; naphthyl unsubstituted or substituted with at least one deuterium; 9,9-dimethylfluorenyl unsubstituted or substituted with at least one deuterium; 9,9-diphenylfluorenyl unsubstituted or substituted with at least one deuterium; triphenylenyl unsubstituted or substituted with at least one deuterium; chrysenyl unsubstituted or substituted with at least one deuterium; dibenzofuranyl unsubstituted or substituted with at least one deuterium; phenyldibenzofuranyl unsubstituted or substituted with at least one deuterium; dibenzothiophenyl unsubstituted or substituted with at least one deuterium; or phenyldibenzothiophenyl unsubstituted or substituted with at least one deuterium.

In the Chemical Formula 1, L₁ and L₂ may be each independently a single bond; substituted or unsubstituted C₆₋₂₀ arylene; or substituted or unsubstituted C₂₋₂₀ heteroarylene containing at least one selected from the group consisting of O, S, Si, P and B; Ar₁ may be cyano; substituted or unsubstituted C₆₋₂₀ aryl; or substituted or unsubstituted C₂₋₂₀ heteroaryl containing at least one heteroatom selected from the group consisting of O, S, Si, P and B; and Ar₂ and Ar₃ may be substituted or unsubstituted C₆₋₂₀ aryl; or substituted or unsubstituted C₂₋₂₀ heteroaryl containing at least one heteroatom selected from the group consisting of O, S, Si, P and B. Preferably, the heteroatom of the heteroarylene or heteroaryl may be O and/or S.

In the Chemical Formula 1, L₁ and L₂ may be each independently a single bond; phenylene unsubstituted or substituted with at least one deuterium; or biphenylylene unsubstituted or substituted with at least one deuterium; and Ar₁ may be cyano; phenyl unsubstituted or substituted with at least one deuterium; biphenylyl unsubstituted or substituted with at least one deuterium; terphenylyl unsubstituted or substituted with at least one deuterium; triphenylenyl unsubstituted or substituted with at least one deuterium; benzofuranyl unsubstituted or substituted with at least one deuterium; benzothiophenyl unsubstituted or substituted with at least one deuterium; dibenzofuranyl unsubstituted or substituted with at least one deuterium; or dibenzothiophenyl unsubstituted or substituted with at least one deuterium; and Ar₂ and Ar₃ may be each independently phenyl unsubstituted or substituted with at least one deuterium; fluorophenyl unsubstituted or substituted with at least one deuterium; cyanophenyl unsubstituted or substituted with at least one deuterium; trimethylsilanophenyl unsubstituted or substituted with at least one deuterium; dimethylphenyl unsubstituted or substituted with at least one deuterium; t-butylphenyl unsubstituted or substituted with at least one deuterium; ethenylphenyl unsubstituted or substituted with at least one deuterium; biphenylyl unsubstituted or substituted with at least one deuterium; terphenylyl unsubstituted or substituted with at least one deuterium; naphthyl unsubstituted or substituted with at least one deuterium; 9,9-dimethylfluorenyl unsubstituted or substituted with at least one deuterium; 9,9-diphenylfluorenyl unsubstituted or substituted with at least one deuterium; triphenylenyl unsubstituted or substituted with at least one deuterium; chrysenyl unsubstituted or substituted with at least one deuterium; dibenzofuranyl unsubstituted or substituted with at least one deuterium; phenyldibenzofuranyl unsubstituted or substituted with at least one deuterium; dibenzothiophenyl unsubstituted or substituted with at least one deuterium; or phenyldibenzothiophenyl unsubstituted or substituted with at least one deuterium.

Representative examples of the compound represented by the Chemical Formula 1 are as follows:

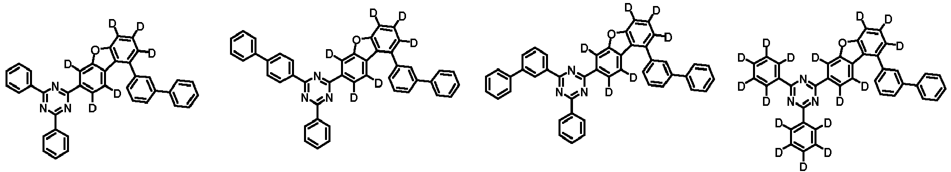

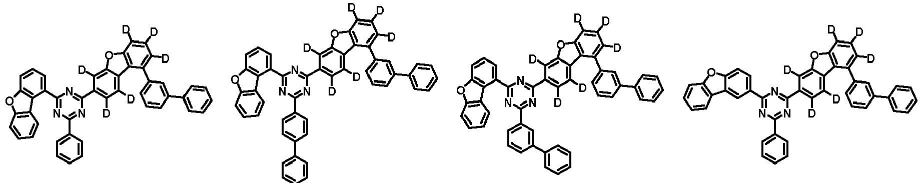

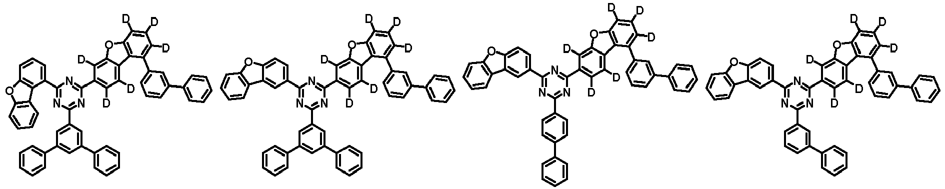

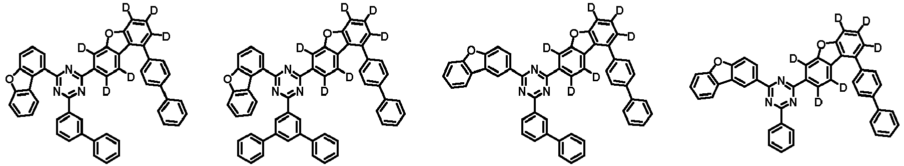

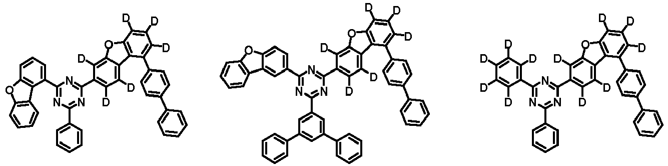

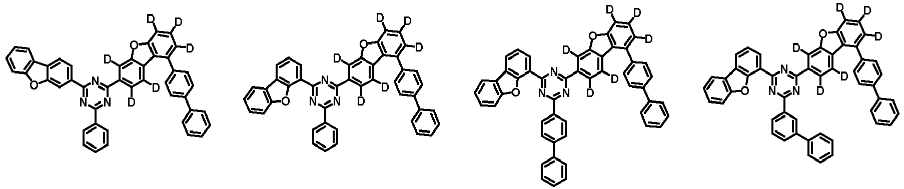

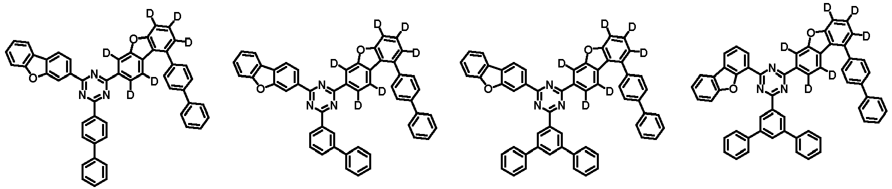

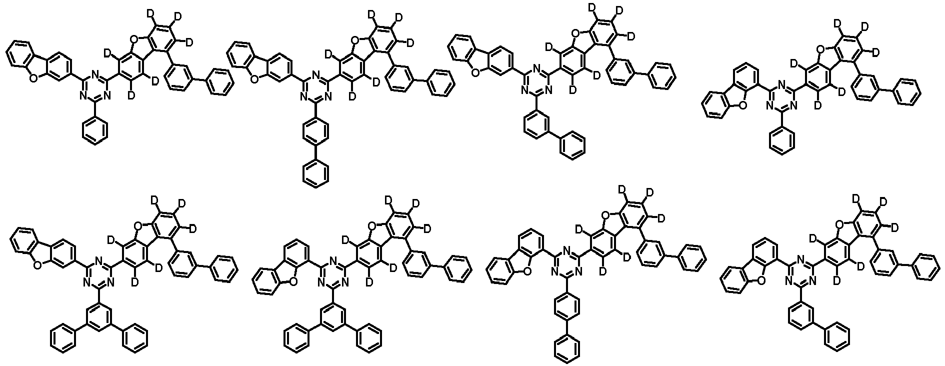

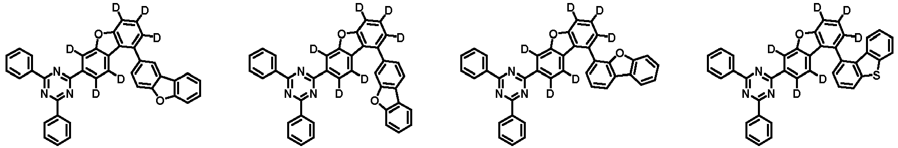

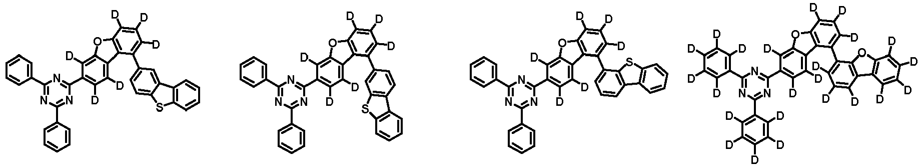

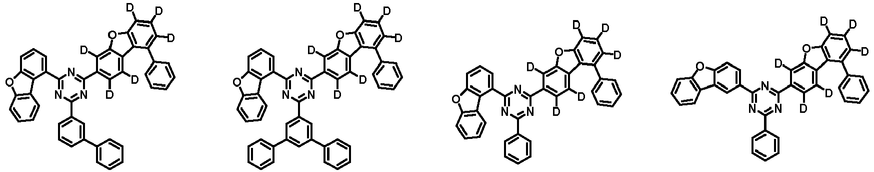

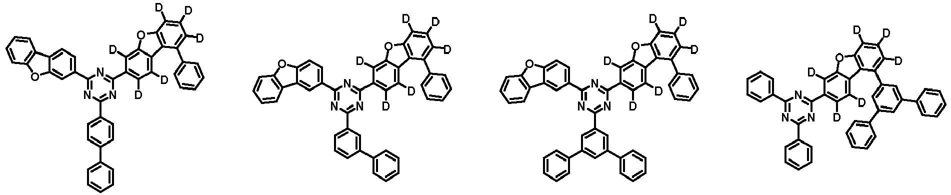

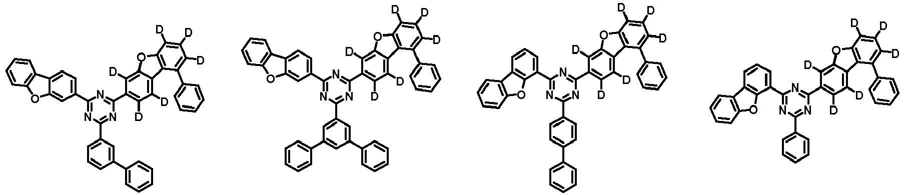

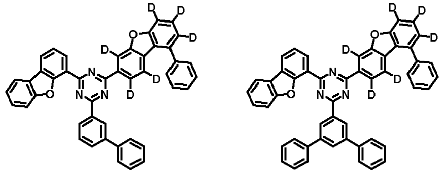

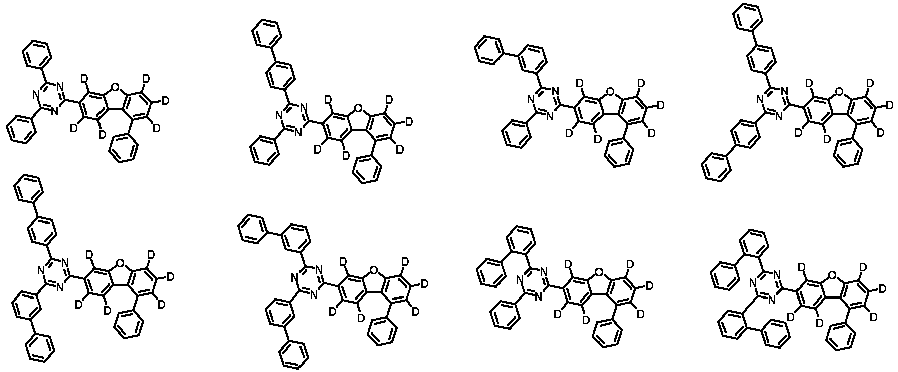

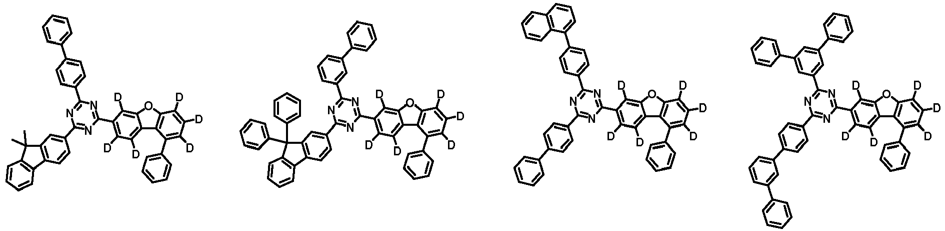

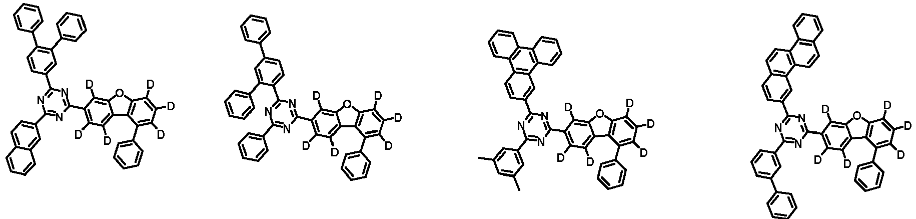

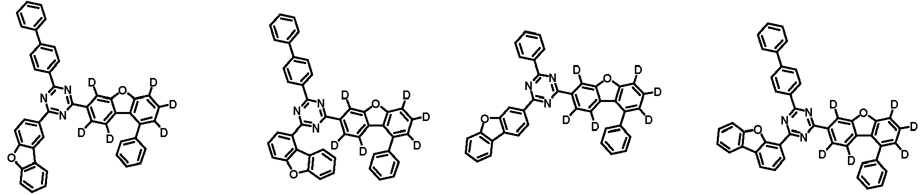

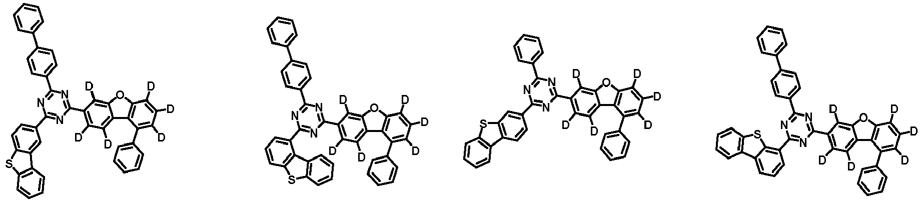

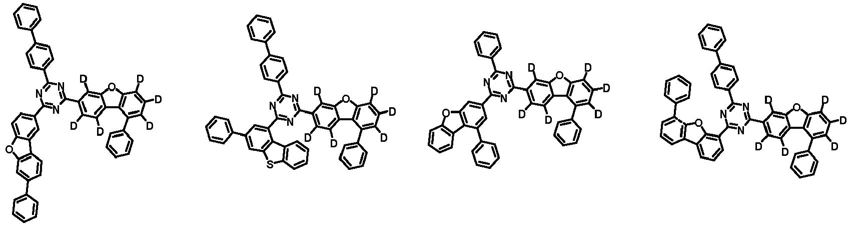

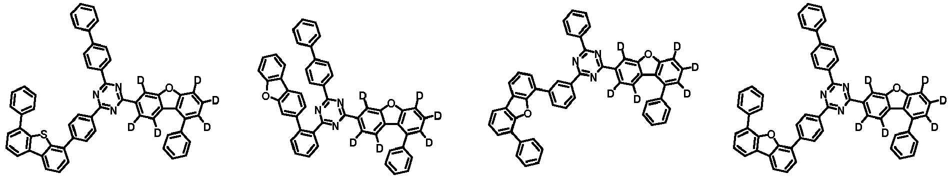

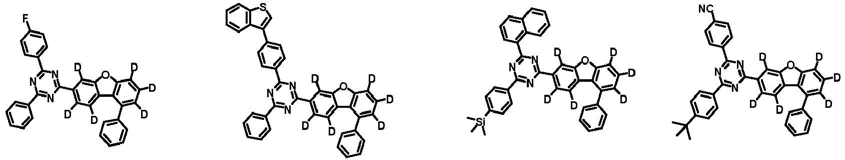

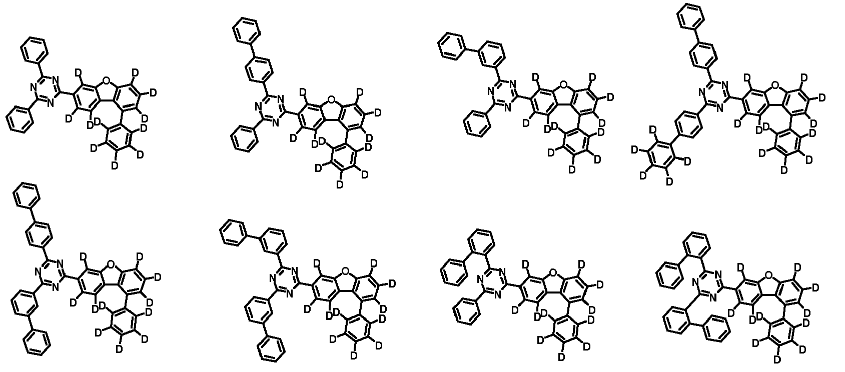

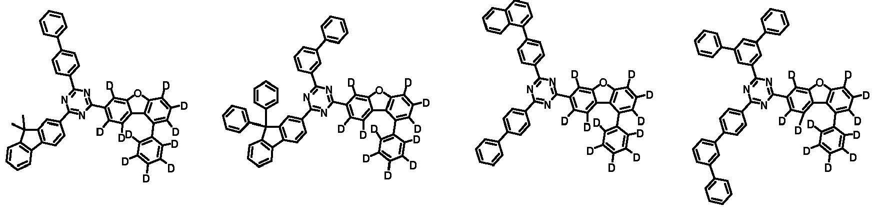

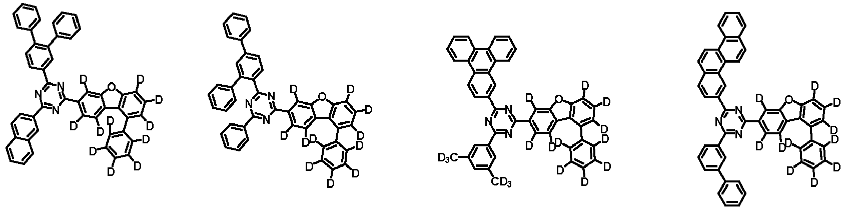

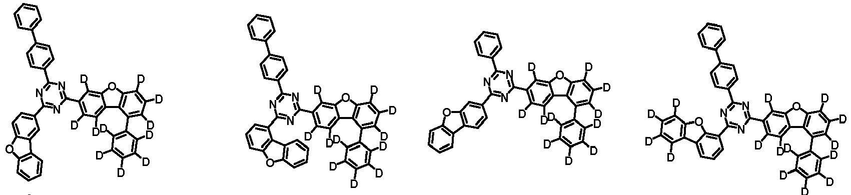

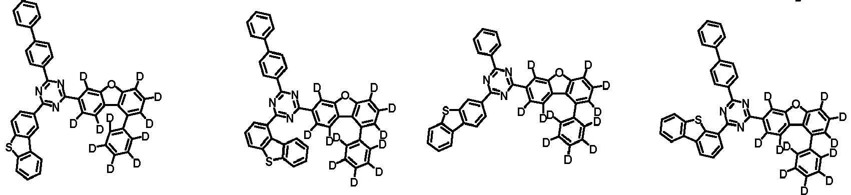

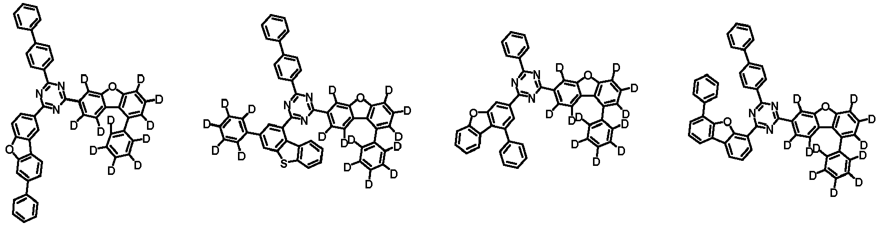

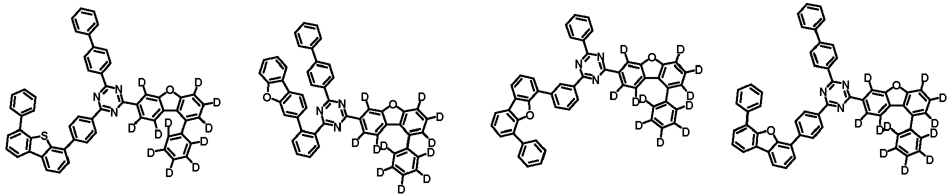

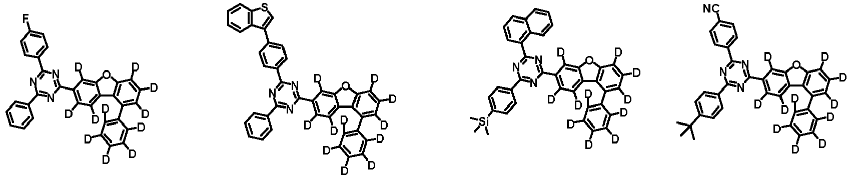

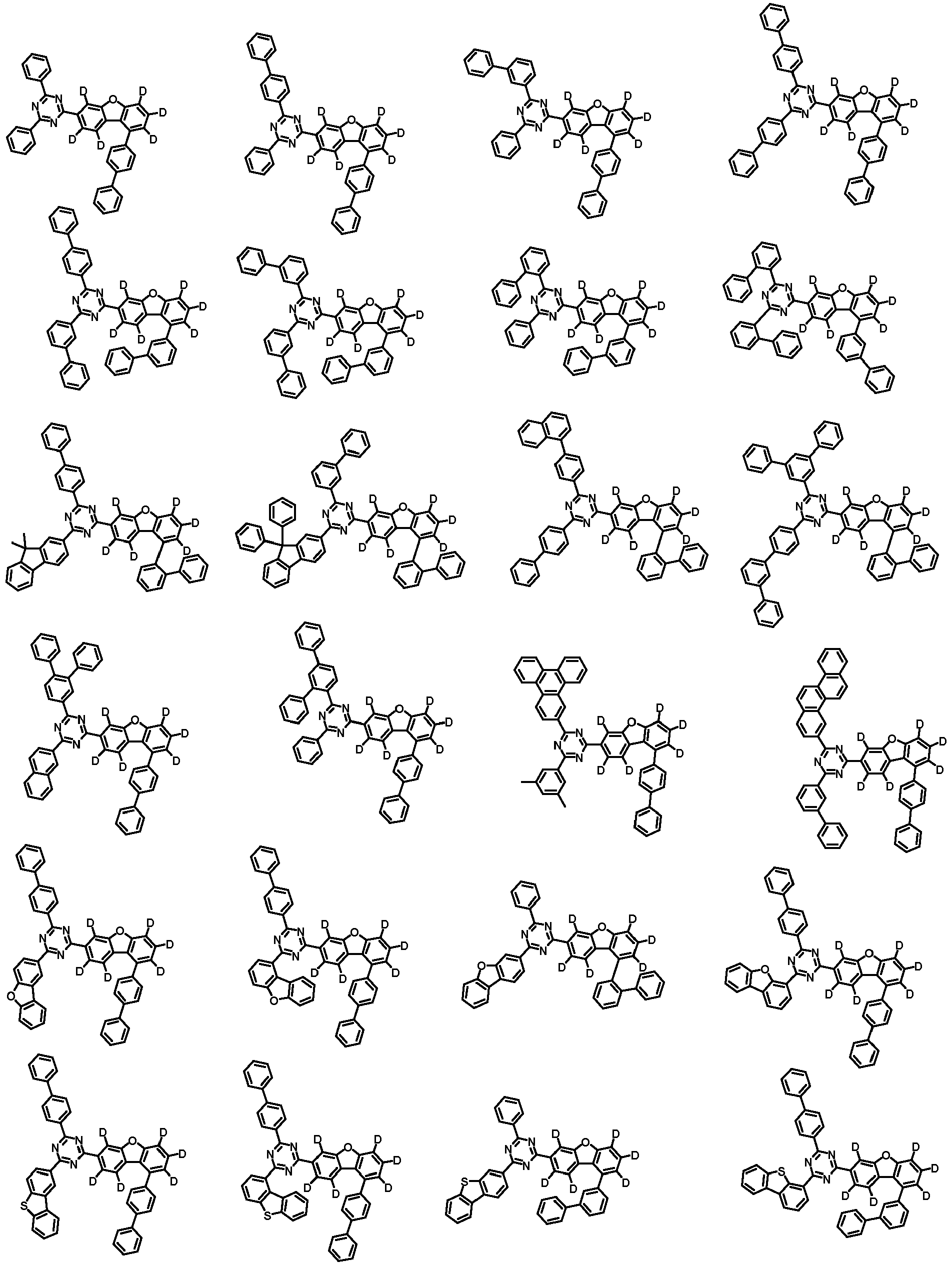

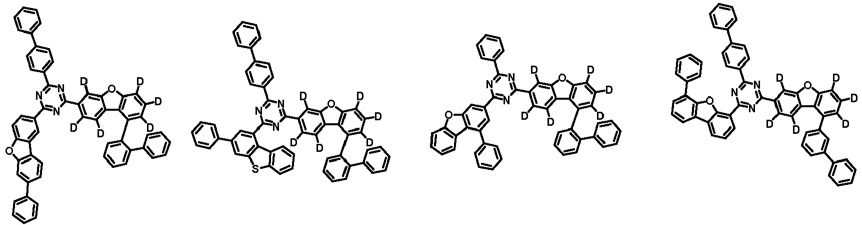

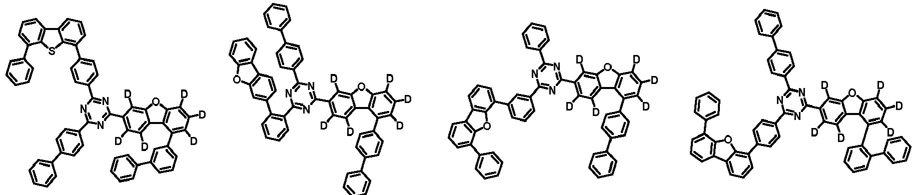

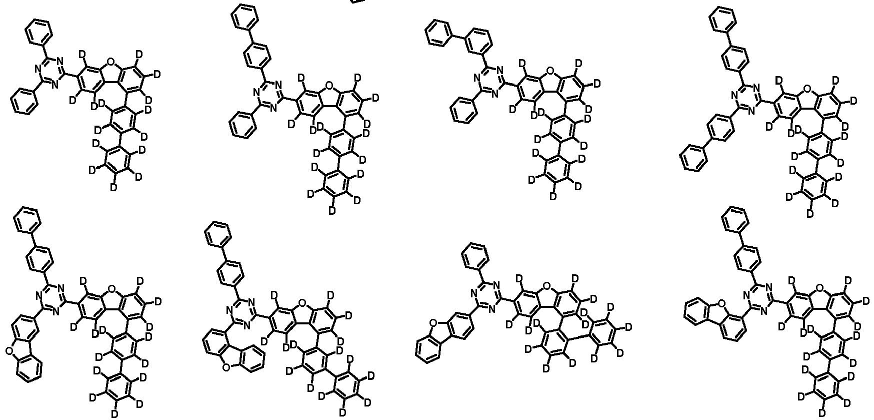

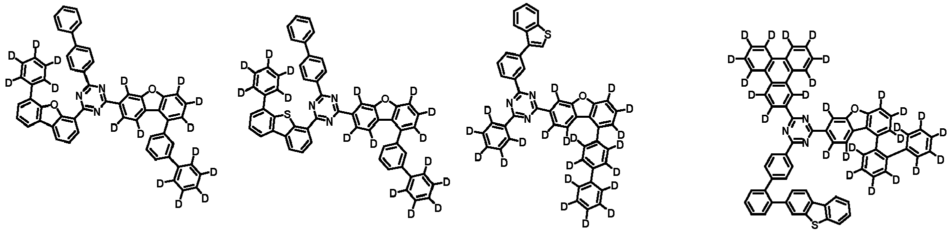

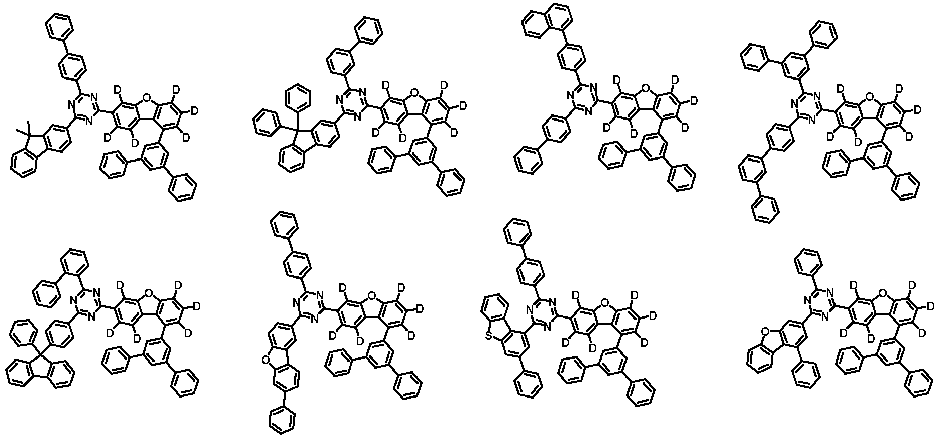

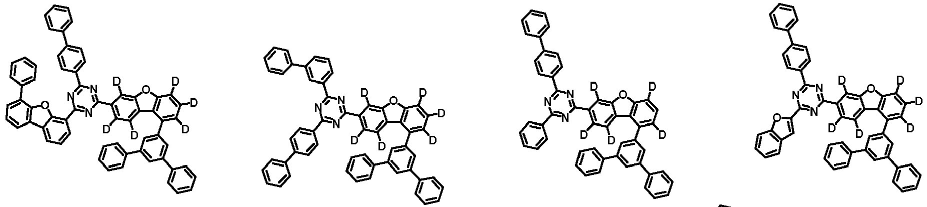

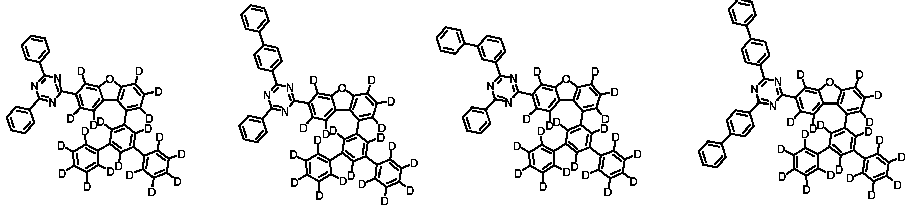

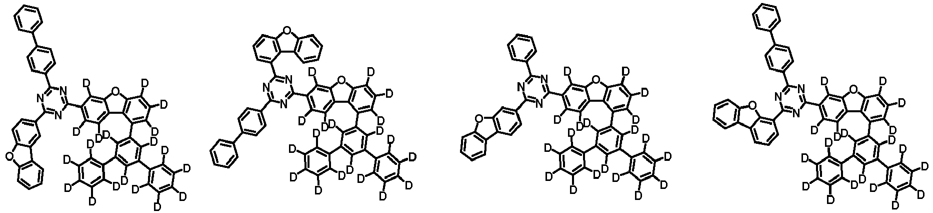

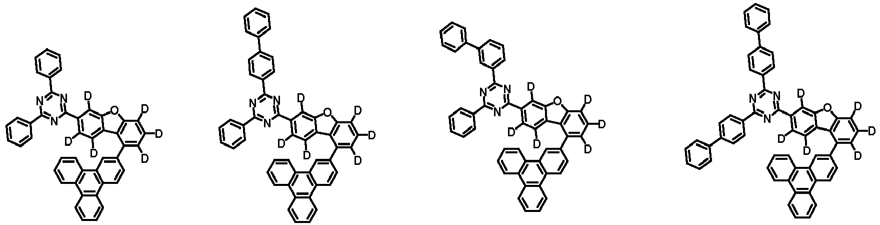

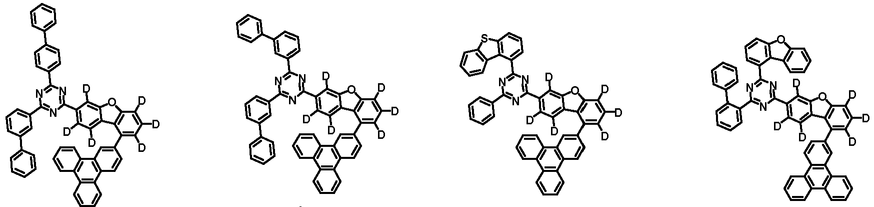

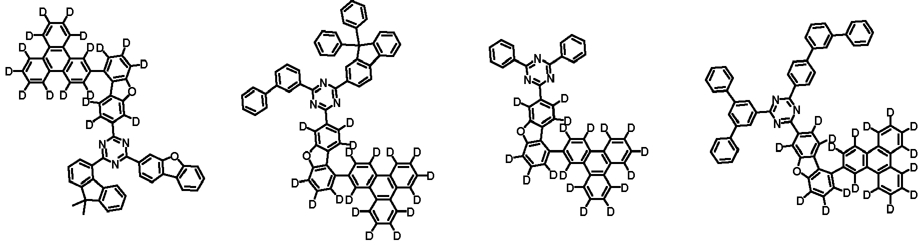

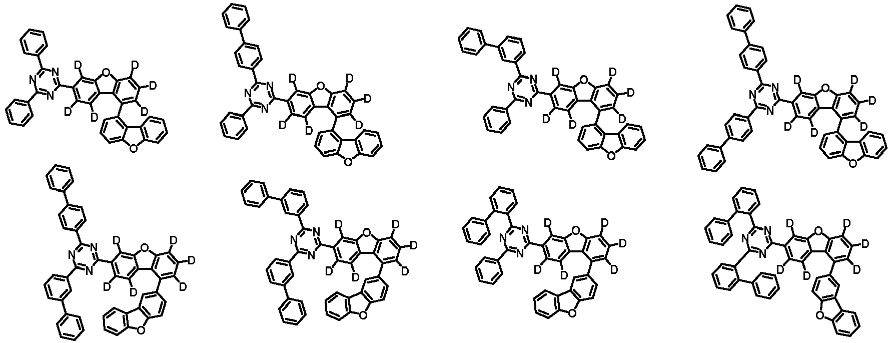

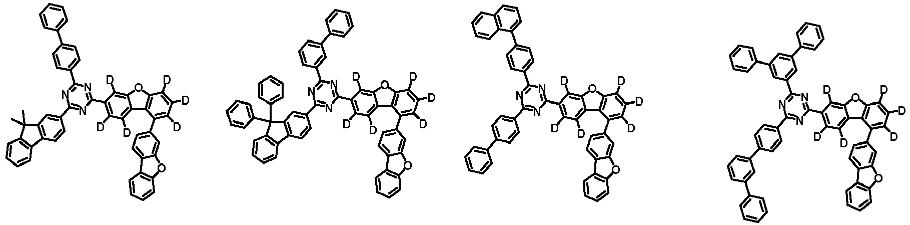

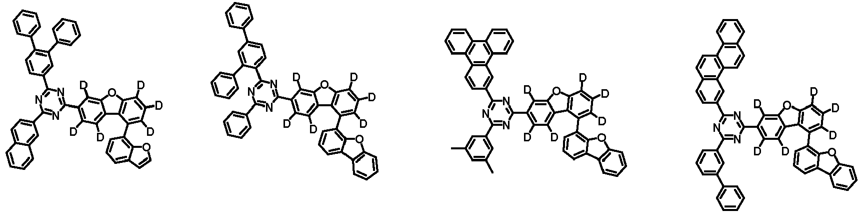

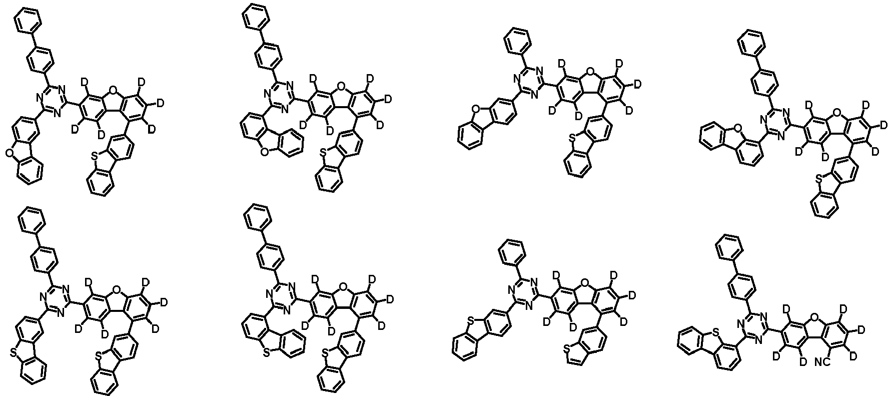

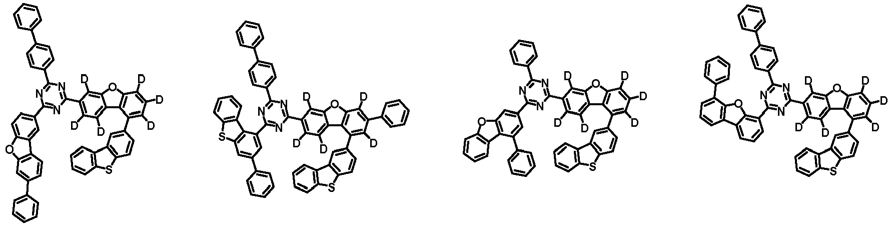

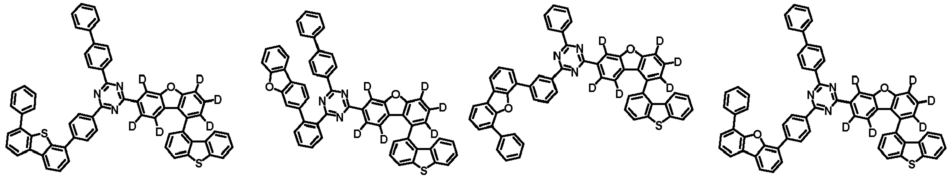

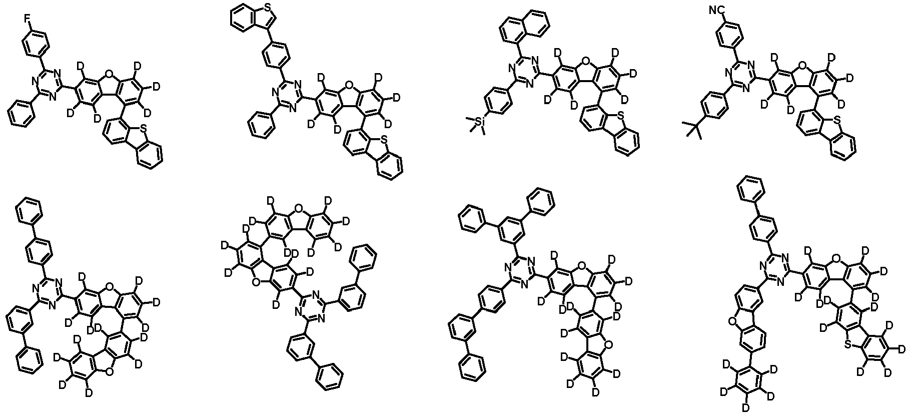

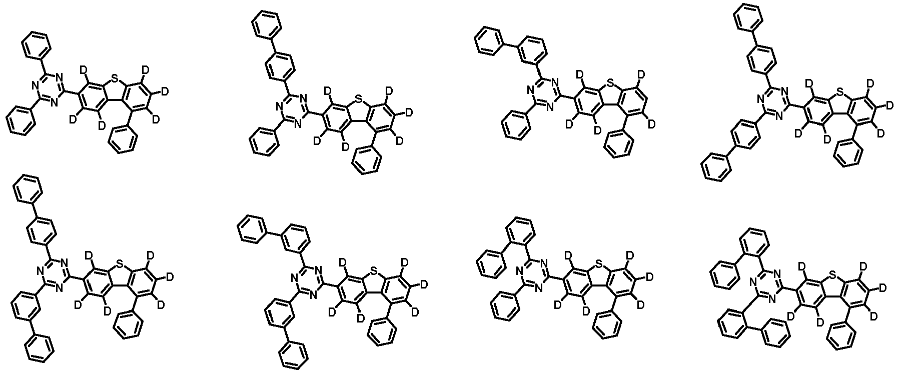

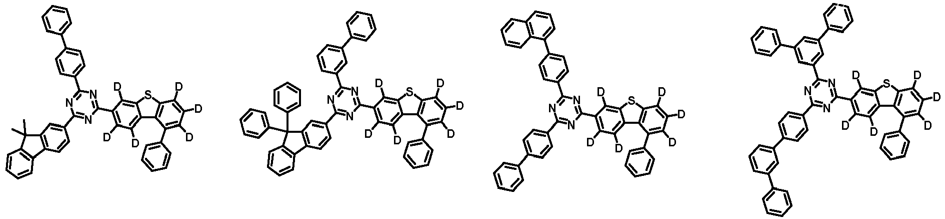

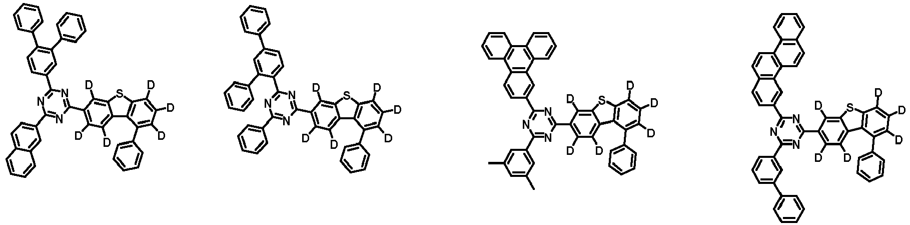

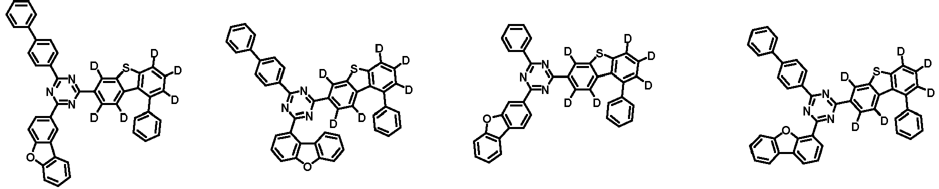

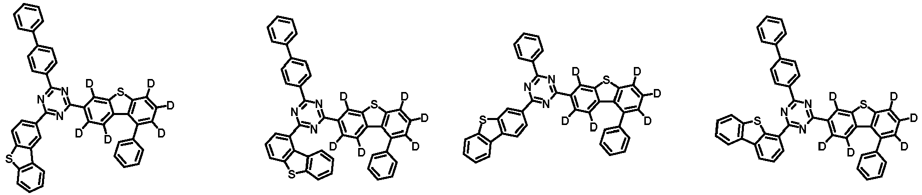

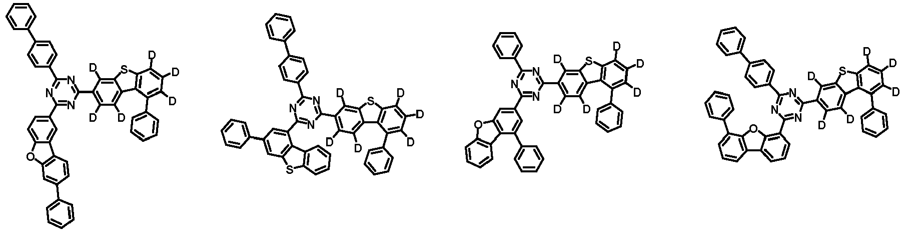

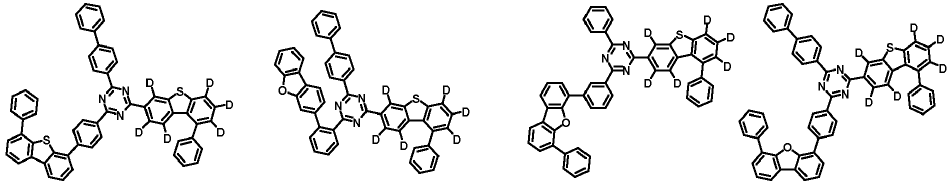

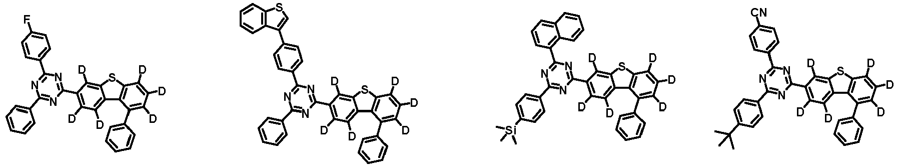

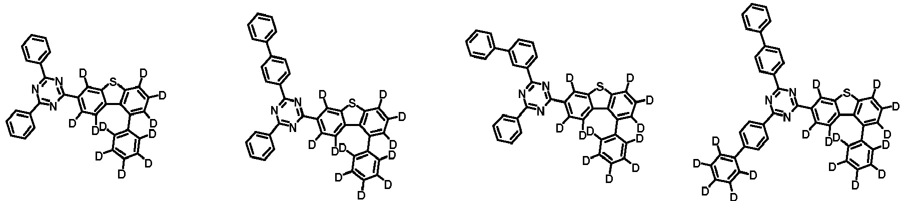

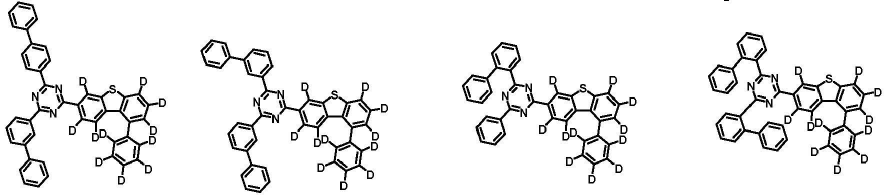

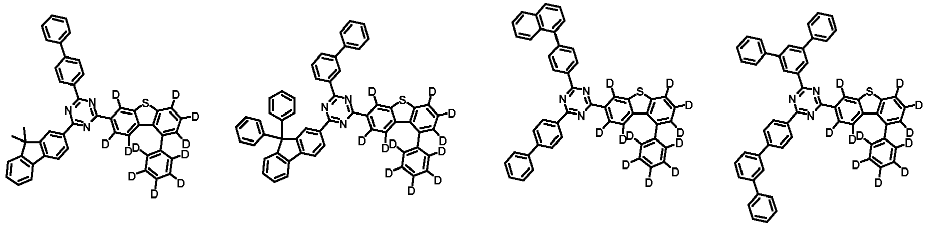

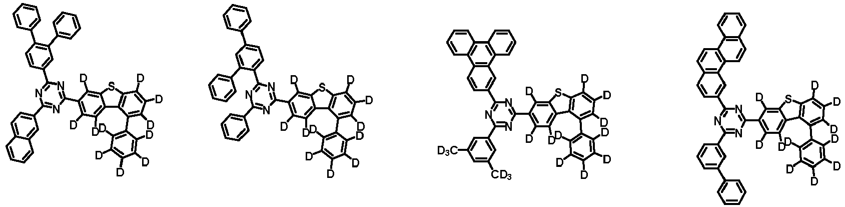

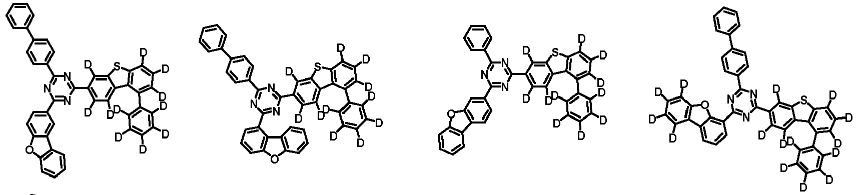

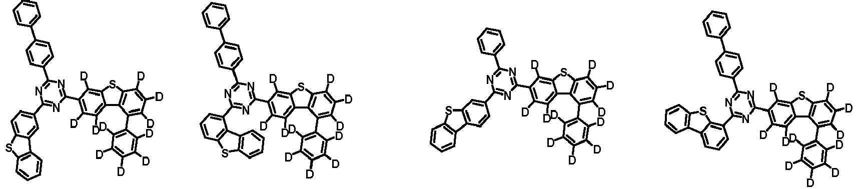

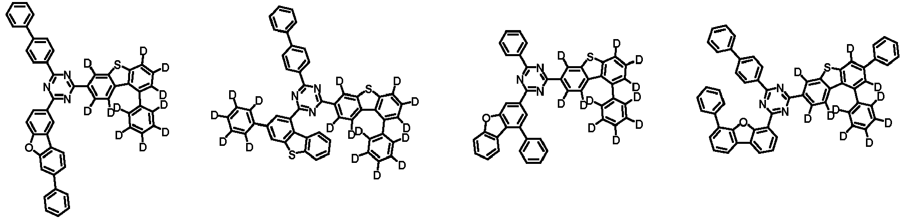

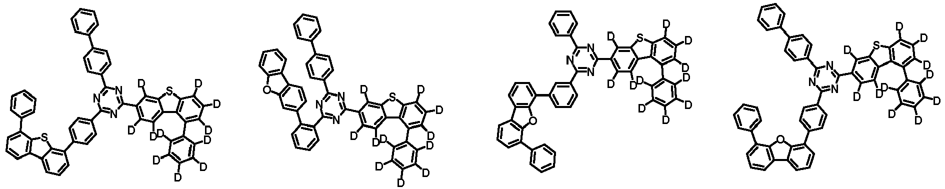

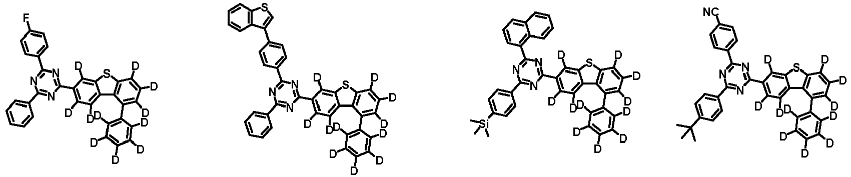

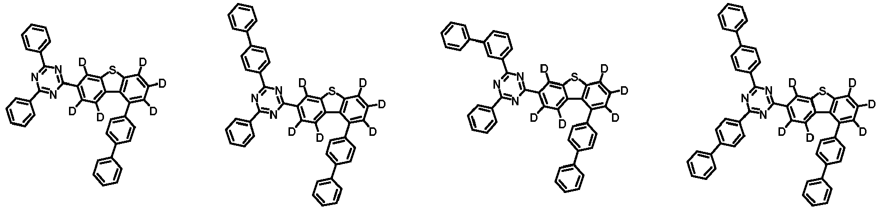

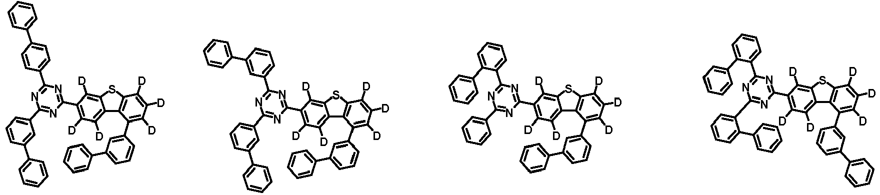

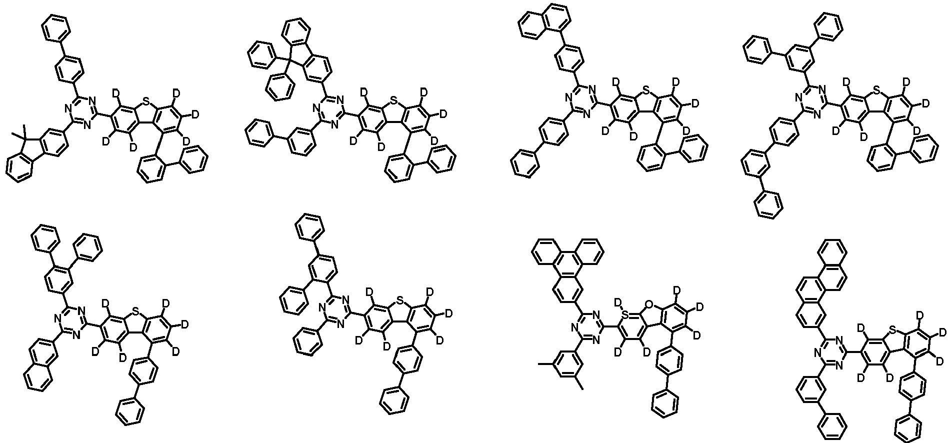

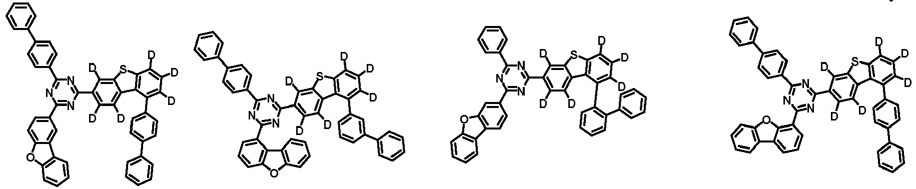

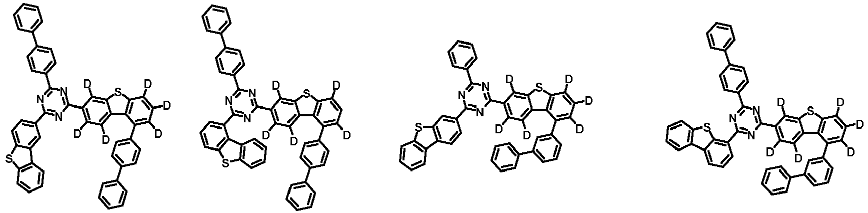

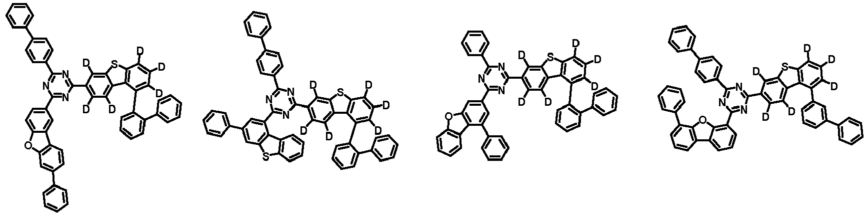

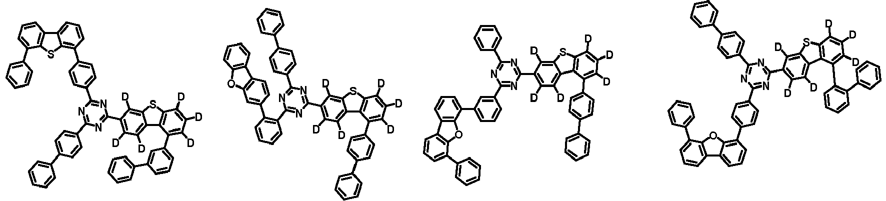

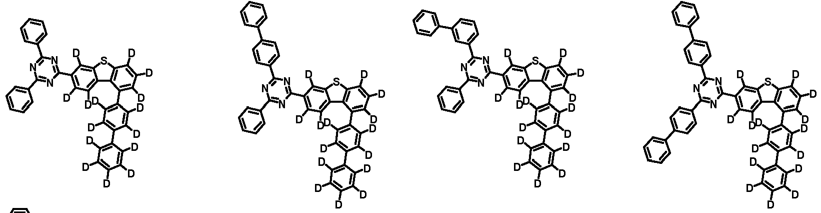

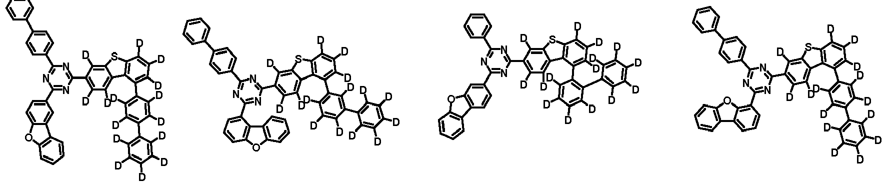

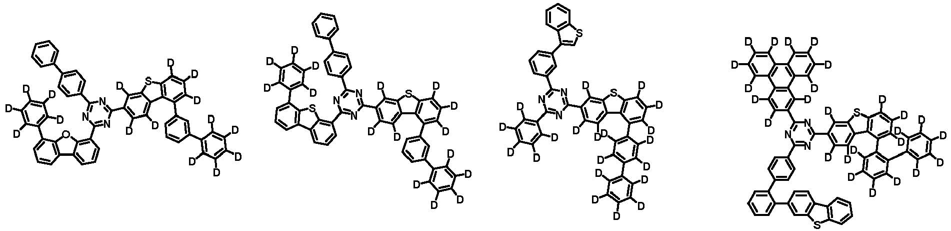

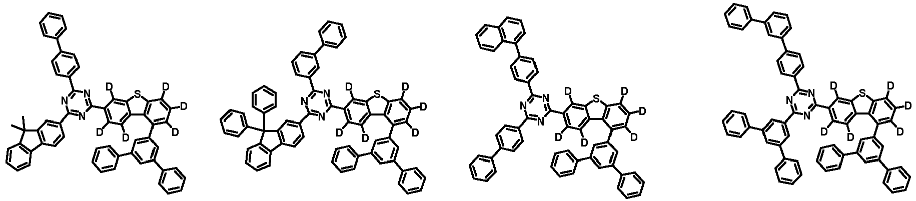

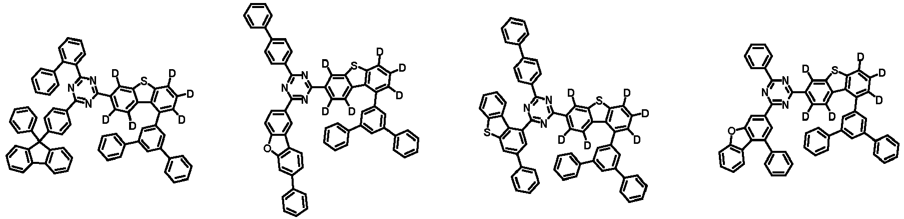

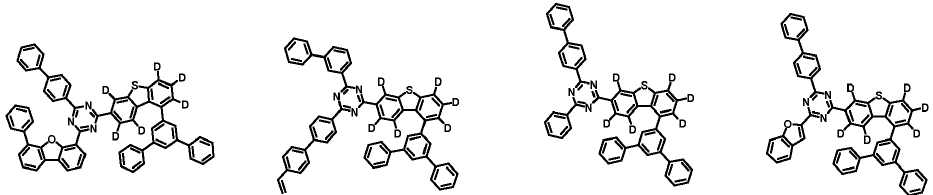

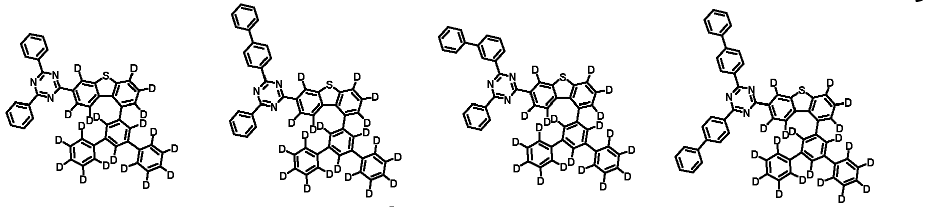

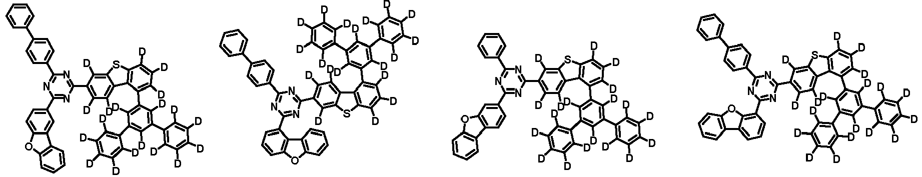

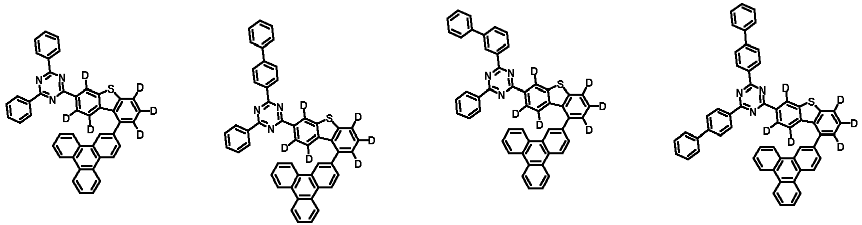

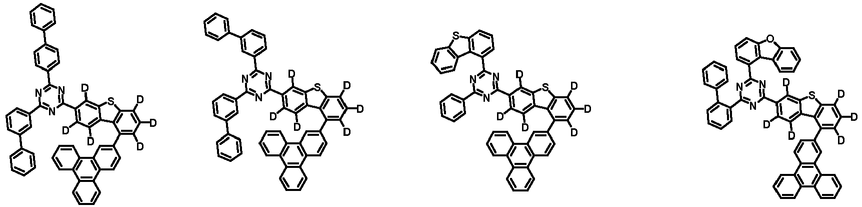

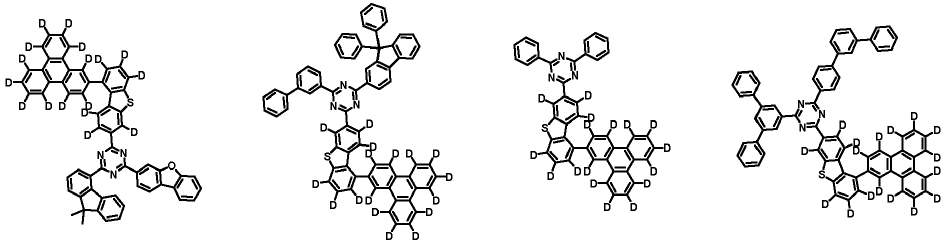

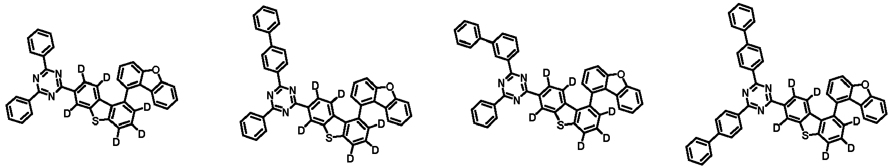

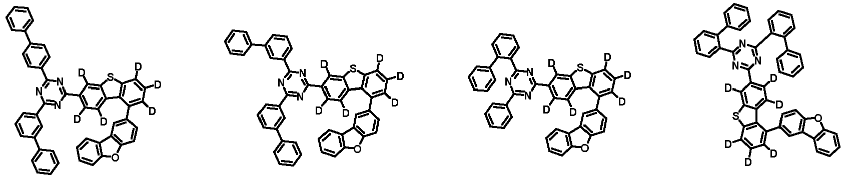

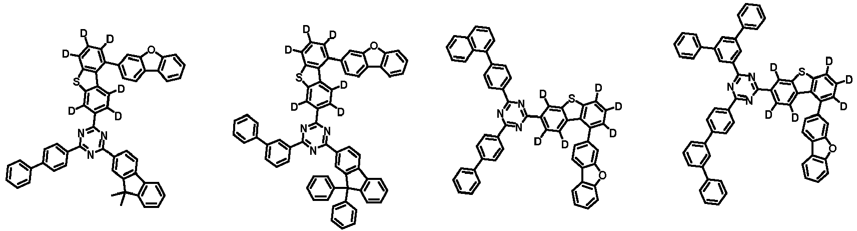

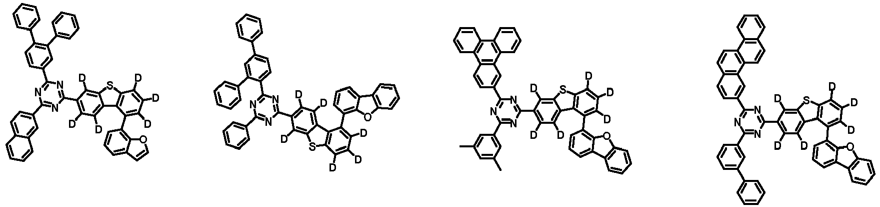

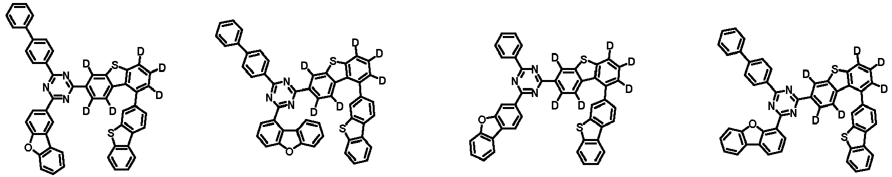

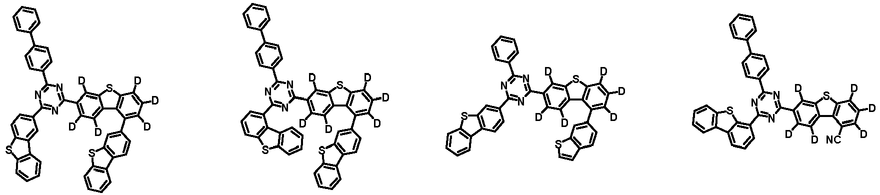

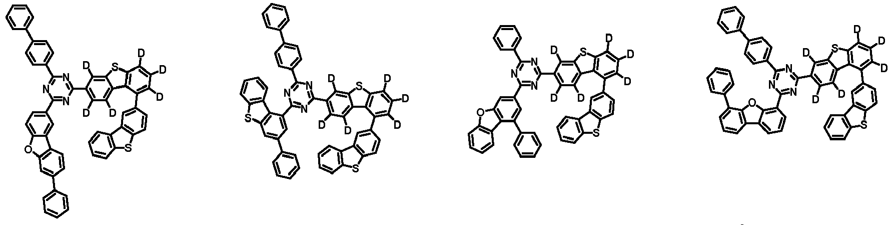

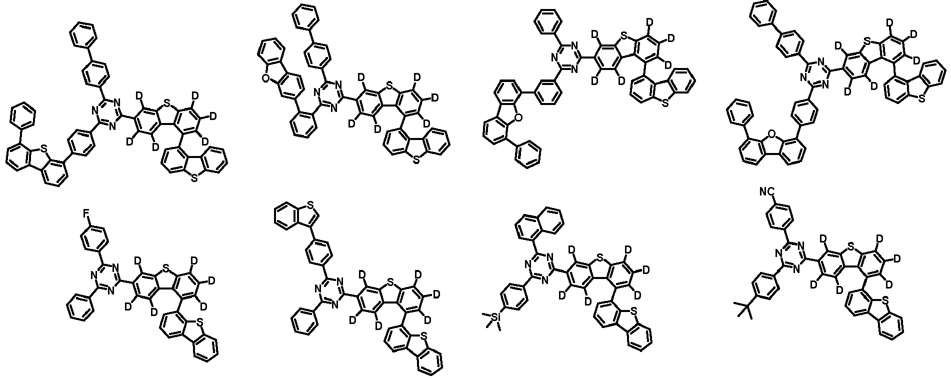

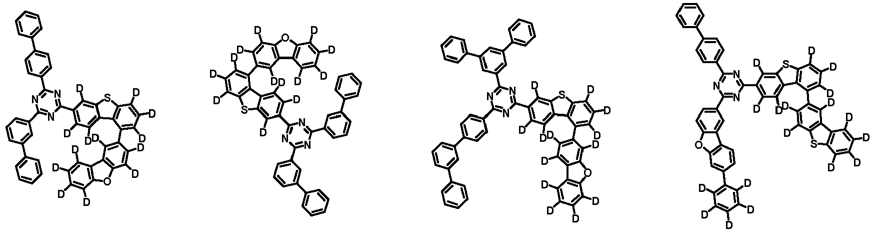

In addition, there is provided a method for preparing a compound represented by the Chemical Formula 1.

For example, the compound represented by the Chemical Formula 1 may be prepared by the preparation method of Reaction Scheme 1 below:

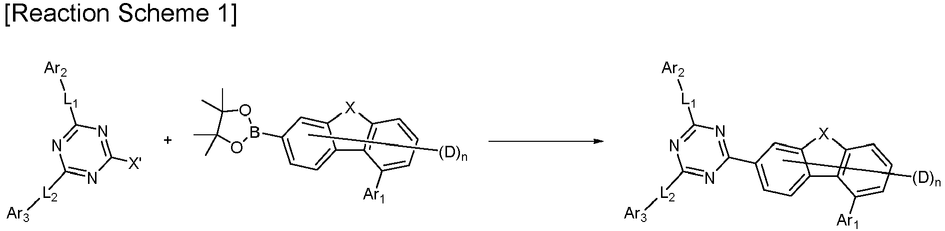

In the Reaction Scheme 1, definitions of other substituents except for X' are the same as defined above, and X' is halogen, preferably bromo, or chloro.

The above reaction is a Suzuki coupling reaction, and preferably performed in the presence of a palladium catalyst and a base, and the reactive group for the reaction may be appropriately changed.

The preparation method may be more specifically described in Preparation Examples described below.

In addition, there is provided an organic light emitting device including the compound represented by the Chemical Formula 1. As an example, there is provided an organic light emitting device including: a first electrode; a second electrode that is provided opposite to the first electrode; and one or more organic material layers that are provided between the first electrode and the second electrode, wherein at least one layer of the organic material layers includes the compound represented by the Chemical Formula 1.

The organic material layer of the organic light emitting device of the present disclosure may have a single-layer structure, or it may have a multilayered structure in which two or more organic material layers are stacked. For example, the organic light emitting device of the present disclosure may have a structure including a hole injection layer, a hole transport layer, a light emitting layer, an electron transport layer, an electron injection layer, and the like as the organic material layer. However, the structure of the organic light emitting device is not limited thereto, and it may include a smaller number of organic layers.

In addition, the organic material layer may include a light emitting layer, and the light emitting layer includes the compound represented by the Chemical Formula 1. In particular, the compound according to the present disclosure can be used as a host in the light emitting layer.

In addition, the organic material layer may include a hole injection layer, a hole transport layer, or an electron blocking layer, and the hole injection layer, hole transport layer, or electron blocking layer includes the compound represented by the Chemical Formula 1.

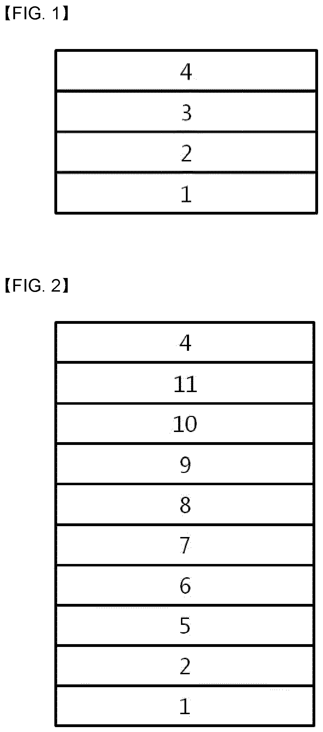

Further, the organic light emitting device according to the present disclosure may be a normal type organic light emitting device in which an anode, one or more organic material layers and a cathode are sequentially stacked on a substrate. Further, the organic light emitting device according to the present disclosure may be an inverted type organic light emitting device in which a cathode, one or more organic material layers and an anode are sequentially stacked on a substrate. For example, the structure of an organic light emitting device according to an embodiment of the present disclosure is illustrated in FIGS. 1 to 3.

FIG. 1 shows an example of an organic light emitting device including a substrate 1, an anode 2, an organic material layer 3, and a cathode 4. In such a structure, the compound represented by the Chemical Formula 1 may be included in the light emitting layer.

FIG. 2 shows an example of an organic light emitting device including a substrate 1, an anode 2, a hole injection layer 5, a hole transport layer 6, an electron blocking layer 7, a light emitting layer 8, a hole blocking layer 9, an electron transport layer 10, an electron injection layer 11, and a cathode 4. In such a structure, the compound represented by the Chemical Formula 1 may be included in one or more layers of the hole injection layer, the hole transport layer, the electron blocking layer, the light emitting layer, the hole blocking layer, the electron transport layer, and the electron injection layer. For example, it may be included in the light emitting layer.

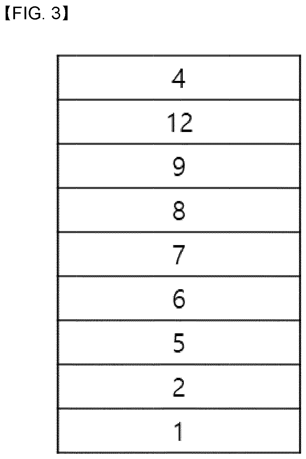

FIG. 3 shows an example of an organic light emitting device including a substrate 1, an anode 2, a hole injection layer 5, a hole transport layer 6, an electron blocking layer 7, a light emitting layer 8, a hole blocking layer 9, an electron injection and transport layer 12, and a cathode 4. In such a structure, the compound represented by the Chemical Formula 1 may be included in one or more layers of the hole injection layer, the hole transport layer, the electron blocking layer, the light emitting layer, the hole blocking layer, and the electron injection and transport layer. For example, it may be included in the light emitting layer.

The organic light emitting device according to the present disclosure may be manufactured using materials and methods known in the art, except that at least one layer of the organic material layers includes the compound represented by the Chemical Formula 1. Moreover, when the organic light emitting device includes a plurality of organic material layers, the organic material layers may be formed of the same material or different materials.

For example, the organic light emitting device according to the present disclosure can be manufactured by sequentially stacking a first electrode, an organic material layer and a second electrode on a substrate. In this case, the organic light emitting device may be manufactured by depositing a metal, metal oxides having conductivity, or an alloy thereof on the substrate using a PVD (physical vapor deposition) method such as a sputtering method or an e-beam evaporation method to form an anode, forming organic material layers including the hole injection layer, the hole transport layer, the light emitting layer and the electron transport layer thereon, and then depositing a material that can be used as the cathode thereon. In addition to such a method, the organic light emitting device may be manufactured by sequentially depositing a cathode material, an organic material layer and an anode material on a substrate.

Further, the compound represented by the Chemical Formula 1 may be formed into an organic material layer by a solution coating method as well as a vacuum deposition method at the time of manufacturing an organic light emitting device. Herein, the solution coating method means a spin coating, a dip coating, a doctor blading, an inkjet printing, a screen printing, a spray method, a roll coating, or the like, but is not limited thereto.

In addition to such a method, the organic light emitting device may be manufactured by sequentially depositing a cathode material, an organic material layer and an anode material on a substrate (International Publication WO2003/012890). However, the manufacturing method is not limited thereto.

For example, the first electrode is an anode, and the second electrode is a cathode, or alternatively, the first electrode is a cathode and the second electrode is an anode.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:Al or SnO₂:Sb; conductive polymers such as poly(3-methylthiophene), poly[3,4-(ethylene-1 ,2-dioxy)thiophene](PEDOT), polypyrrole, and polyaniline, and the like, but are not limited thereto.

As the cathode material, generally, a material having a small work function is preferably used so that electrons can be easily injected into the organic material layer.

Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

The hole injection layer is a layer for injecting holes from the electrode, and the hole injection material is preferably a compound which has a capability of transporting the holes, thus has a hole injecting effect in the anode and an excellent hole-injecting effect to the light emitting layer or the light emitting material, prevents excitons produced in the light emitting layer from moving to an electron injection layer or the electron injection material, and is excellent in the ability to form a thin film. It is preferable that a HOMO (highest occupied molecular orbital) of the hole injection material is between the work function of the anode material and a HOMO of a peripheral organic material layer. Specific examples of the hole injection material include metal porphyrine, oligothiophene, an arylamine-based organic material, a hexanitrilehexaazatriphenylene-based organic material, a quinacridone-based organic material, a perylene-based organic material, anthraquinone, polyaniline and polythiophene-based conductive polymer, and the like, but are not limited thereto.

In addition, the hole transport layer is a layer that receives holes from a hole injection layer and transports the holes to the light emitting layer. The hole transport material is suitably a material having large mobility to the holes, which may receive holes from the anode or the hole injection layer and transfer the holes to the light emitting layer. Specific examples thereof include an arylamine-based organic material, a conductive polymer, a block copolymer in which a conjugate portion and a non-conjugate portion are present together, and the like, but are not limited thereto.

The electron blocking layer prevents electrons injected from the cathode from being transferred to the anode without recombination in the light emitting layer, thereby improving the efficiency of the organic light emitting device.

The light emitting material is suitably a material capable of emitting light in a visible ray region by receiving holes and electrons from the hole transport layer and the electron transport layer, respectively, to combine them, and having good quantum efficiency to fluorescence or phosphorescence. Specific examples thereof include 8-hydroxyquinoline aluminum complex (Alq₃); a carbazole-based compound; a dimerized styryl compound; BAlq; a 10-hydroxybenzo quinoline-metal compound; a benzoxazole-, benzthiazole- and benzimidazole-based compound; a poly(p-phenylenevinylene) (PPV)-based polymer; a spiro compound; polyfluorene, rubrene, and the like, but are not limited thereto.

In addition, the light emitting layer may include a host material and a dopant material. The host material may be a fused aromatic ring derivative or a heterocycle-containing compound. Specific examples of the fused aromatic ring derivatives include anthracene derivatives, pyrene derivatives, naphthalene derivatives, pentacene derivatives, phenanthrene compounds, fluoranthene compounds, and the like. Examples of the heterocyclic-containing compounds include carbazole derivatives, dibenzofuran derivatives, ladder-type furan compounds, pyrimidine derivatives, and the like, but are not limited thereto. In particular, in the present disclosure, the compound represented by the Chemical Formula 1 may be used as a host material of the light emitting layer, and in this case, the organic light emitting device may have low voltage, high efficiency and/or long lifespan.

The dopant material includes an aromatic amine derivative, a styrylamine compound, a boron complex, a fluoranthene compound, a metal complex, and the like. Specifically, the aromatic amine derivative is a substituted or unsubstituted fused aromatic ring derivative having an arylamino group, and examples thereof include pyrene, anthracene, chrysene, periflanthene and the like, which have an arylamino group. The styrylamine compound is a compound where at least one arylvinyl group is substituted in substituted or unsubstituted arylamine, in which one or two or more substituent groups selected from the group consisting of an aryl group, a silyl group, an alkyl group, a cycloalkyl group, and an arylamino group are substituted or unsubstituted. Specific examples thereof include styrylamine, styryldiamine, styryltriamine, styryltetramine, and the like, but are not limited thereto. Further, the metal complex includes an iridium complex, a platinum complex, and the like, but is not limited thereto.

The electron transport layer is a layer which receives electrons from an electron injection layer and transports the electrons to a light emitting layer, and an electron transport material used is suitably a material which can receive electrons well from a cathode and transfer the electrons to a light emitting layer and has large mobility for electrons. Specifically, examples thereof may include an Al complex of 8-hydroxyquinoline; a complex including Alqs; an organic radical compound; a hydroxyflavone-metal complex, and the like, but are not limited thereto. The electron transport layer may be used with any desired cathode material, as used according to the related art. In particular, appropriate examples of the cathode material are a typical material which has a low work function, followed by an aluminum layer or a silver layer. Specific examples thereof include cesium, barium, calcium, ytterbium, and samarium, in each case followed by an aluminum layer or a silver layer.

The electron injection layer is a layer which injects electrons from an electrode, and is preferably a compound which has a capability of transporting electrons, has an effect of injecting electrons from a cathode and an excellent effect of injecting electrons into a light emitting layer or a light emitting material, prevents excitons produced from the light emitting layer from moving to a hole injection layer, and is also excellent in the ability to form a thin film. Specific examples thereof include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like, but are not limited thereto.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

According to an embodiment of the present disclosure, the electron injection and transport layer may be formed as a single layer by simultaneously depositing the electron transport material and the electron injection material.

The organic light emitting device according to the present disclosure may be a front side emission type, a backside emission type, or a double-sided emission type according to the used material.

In addition, the compound represented by the Chemical Formula 1 may be included in an organic solar cell or an organic transistor in addition to an organic light emitting device.

The preparation of the compound represented by the Chemical Formula 1 and the organic light emitting device including the same will be described in detail in the following examples. However, these examples are presented for illustrative purposes only, and are not intended to limit the scope of the present disclosure.

### [Preparation Examples]

### Preparation Example 1: Preparation of Compound 1

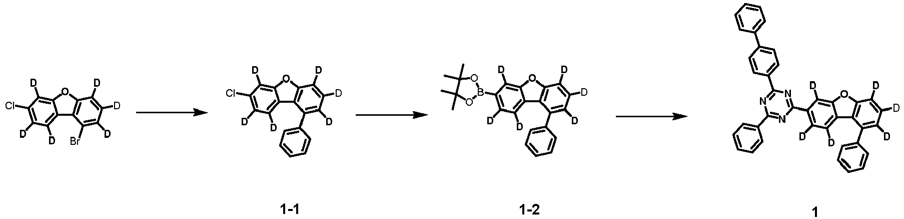

### (Preparation Example 1-1) Preparation of Intermediate 1-1

Under a nitrogen atmosphere, 1-bromo-7-chlorodibenzo[b,d]furan-2,3,4,6,8,9-d6 (50 g, 173.9 mmol) and phenylboronic acid (21.2 g, 173.9mmol) were added to 300ml of THF, stirred and refluxed. Potassium carbonate (72.1 g, 521.6 mmol) was then dissolved in 100 ml of water, stirred thoroughly, and tetrakistriphenyl-phosphinopalladium (6 g, 5.2 mmol) was added. After 2 hours of reaction, the organic layer and the water layer were separated after cooling to room temperature, and the organic layer was distilled. It was again added to 500 mL of chloroform to dissolve, washed twice with water, the organic layer was separated, Magnesium sulfate anhydrate was added, stirred, filtered and the filtrate was distilled under reduced pressure. The concentrated compound was recrystallized from chloroform and ethyl acetate to afford Intermediate 1-1 (25.7 g, 52%, MS: [M+H]+ = 285.8).

### (Preparation Example 1-2) Preparation of Intermediate 1-2

Under a nitrogen atmosphere, Intermediate 1-1 (50 g, 175.6 mmol) and bis(pinacolato)diboron (45 g, 193.1mmol) were added to 350ml of dioxane, stirred and refluxed. Potassium acetate (50.6 g, 526.7 mmol) was then added and stirred thoroughly, followed by palladium dibenzylideneacetonitrate (3 g, 5.3 mmol) and tricyclohexylphosphine (3 g, 10.5 mmol). After a 7-hour reaction, the reaction was cooled to room temperature and the organic layer was filtered to remove salts, and the filtered organic layer was distilled. This was again added to 450 mL of chloroform to dissolve, washed twice with water, the organic layer was separated, Magnesium sulfate anhydrate was added, stirred, filtered and the filtrate was distilled under reduced pressure. The concentrated compound was recrystallized from chloroform and ethanol to afford Intermediate 1-2 (56.8 g, 86%, MS: [M+H]+ = 377.3).

### (Preparation Example 1-3) Preparation of Compound 1

Under a nitrogen atmosphere, Intermediate 1-2 (30 g, 79.7 mmol) and 2-([1,1'-biphenyl]-4-yl)-4-chloro-6-phenyl-1,3,5-triazine (27.4 g, 79.7 mmol) were added to 600 ml of tetrahydrofuran and stirred and refluxed. Potassium carbonate (33.1 g, 239.2 mmol) was then dissolved in 33 ml of water, stirred thoroughly, and tetrakistriphenyl-phosphinopalladium (2.8 g, 2.4 mmol) was added. After a 1 h reaction, the resulting solid was cooled to room temperature and filtered. The solid was dissolved in 1500 mL of chloroform, washed twice with water, the organic layer was separated, Magnesium sulfate anhydrate was added, stirred, filtered and the filtrate was distilled under reduced pressure. The concentrated compound was recrystallized from chloroform and ethyl acetate to afford compound 1 (22.2 g, 50%, MS: [M+H]+ = 558.7).

### Preparation Example 2: Preparation of Compound 2

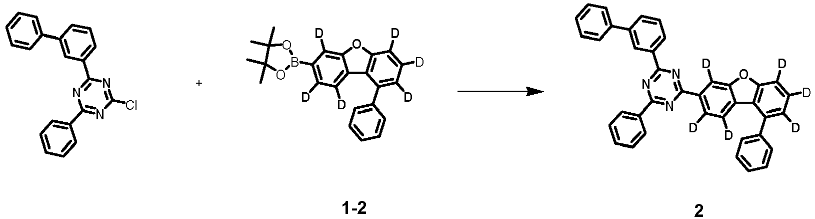

Compounds 1-2 were prepared using the same method as for compound 1, except that 2-([1,1'-biphenyl]-3-yl)-4-chloro-6-phenyl-1,3,5-triazine was used instead of 2-([1,1'-biphenyl]-4-yl)-4-chloro-6-phenyl-1,3,5-triazine. (25.3 g, 57%, MS: [M+H]+ = 558.7)

### Preparation Example 3: Preparation of Compound 3

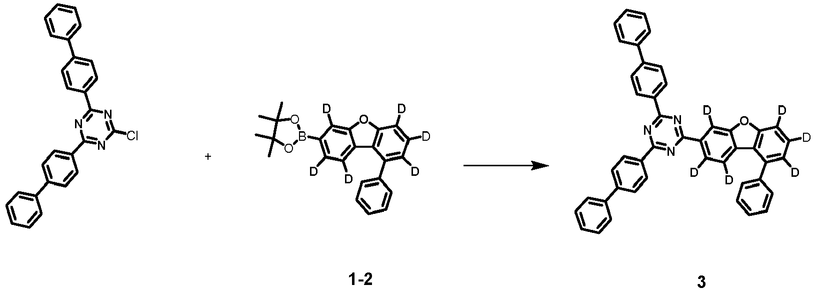

Compound 3 was prepared using the same method as for compound 1, except that 2,4-di([1,1'-biphenyl]-4-yl)-6-chloro-1,3,5-triazine was used instead of 2-([1,1'-biphenyl]-4-yl)-4-chloro-6-phenyl-1,3,5-triazine. (26.3 g, 52%, MS: [M+H]+ = 634.8)

### Preparation Example 4: Preparation of Compound 4

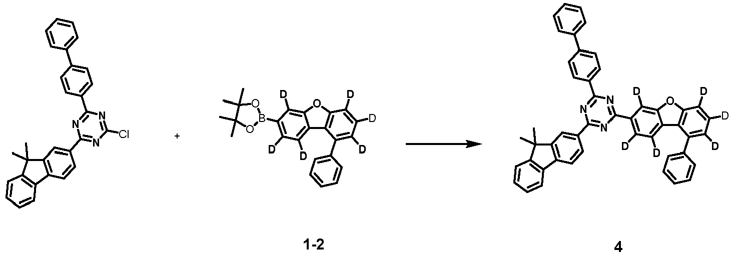

Compound 4 was prepared using the same method as for compound 1, except that 2-([1,1'-biphenyl]-4-yl)-4-chloro-6-(9,9-dimethyl-9H-fluoren-2-yl)-1,3,5-triazine was used instead of 2-([1,1'-biphenyl]-4-yl)-4-chloro-6-phenyl-1,3,5-triazine. (28.1 g, 64%, MS:
[M+H]+ = 674.9)

### Preparation Example 5: Preparation of Compound 5

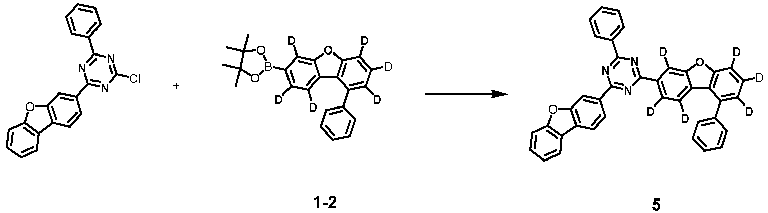

Compound 5 was prepared using the same method as for compound 1, except that 2-chloro-4-(dibenzo[b,d]furan-3-yl)-6-phenyl-1,3,5-triazine was used instead of2-([1,1'-biphenyl]-4-yl)-4-chloro-6-phenyl-1,3,5-triazine. (32.1 g, 67%, MS: [M+H]+ = 572.7)

### Preparation Example 6: Preparation of Compound 6

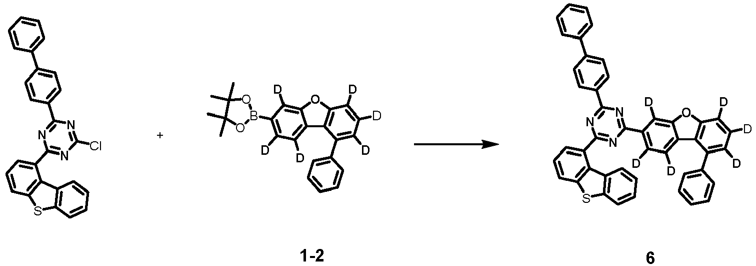

Compound 6 was prepared using the same method as for compound 1, except that 2-([1,1'-biphenyl]-4-yl)-4-chloro-6-(dibenzo[b,d]thiophen-1-yl)-1,3,5-triazine was used instead of 2-([1,1'-biphenyl]-4-yl)-4-chloro-6-phenyl-1,3,5-triazine. (27.4 g, 62%, MS:
[M+H]+ = 664.8)

### Preparation Example 7: Preparation of Compound 7

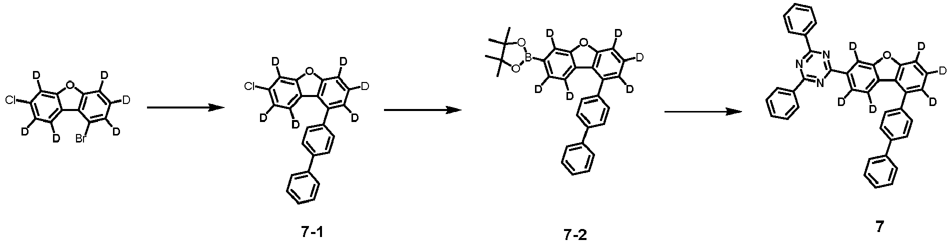

### (Preparation Example 7-1) Preparation of Intermediate 7-1

Intermediate 7-1 was prepared using the same method as for Intermediate 1-1, except that [1,1'-biphenyl]-4-ylboronic acid was used instead of phenylboronic acid. (45.2 g, 72%, MS: [M+H]+ = 361.9)

### (Preparation Example 7-2) Preparation of Intermediate 7-2

Intermediate 7-2 was prepared using the same method as for Intermediate 1-2. (55 g, 88%, MS: [M+H]+ = 452.4)

### (Preparation Example 7-3) Preparation of Compound 7

Compound 7 was prepared using the same method as for the preparation of Compound 1, except that Intermediate 7-2 was used instead of Intermediate 1-2 and 2-chloro-4,6-diphenyl-1,3,5-triazine was used instead of 2-([1,1'-biphenyl]-4-yl)-4-chloro-6-phenyl-1,3,5-triazine. (21.8 g, 59%, MS: [M+H]+ = 558.7)

### Preparation Example 8: Preparation of Compound 8

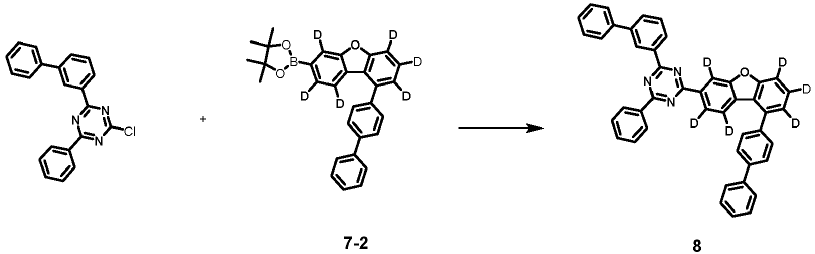

Compound 8 was prepared using the same method as for compound 7, except that 2-([1,1'-biphenyl]-3-yl)-4-chloro-6-phenyl-1,3,5-triazine was used instead of 2-chloro-4,6-diphenyl-1,3,5-triazine. (38.7 g, 70%, MS: [M+H]+ = 634.8)

### Preparation Example 9: Preparation of Compound 9

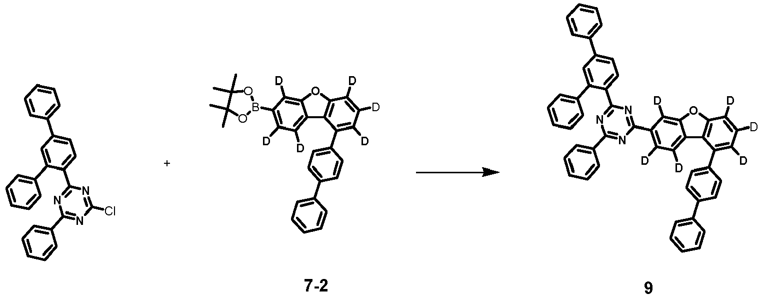

Compound 9 was prepared using the same method as for compound 7, except that 2-([1,1':3',1"-terphenyl]-4'-yl)-4-chloro-6-phenyl-1,3,5-triazine was used instead of 2-chloro-4,6-diphenyl-1,3,5-triazine. (27.4 g, 54%, MS: [M+H]+ = 710.9)

### Preparation Example 10: Preparation of Compound 10

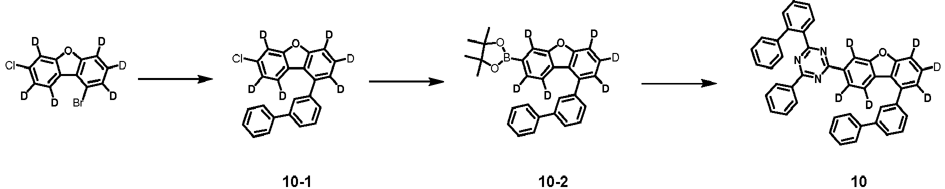

### (Preparation Example 10-1) Preparation of Intermediate 10-1

Intermediate 10-1 was prepared by the same method as the preparation of Intermediate 7-1, except that [1,1'-biphenyl]-3-ylboronic acid was used instead of [1,1'-biphenyl]-4-ylboronic acid. (45.2 g, 72%, MS: [M+H]+ = 361.9)

### (Preparation Example 10-2) Preparation of Intermediate 10-2

Intermediate 10-2 was prepared by synthesizing using the same method as the preparation of Intermediate 7-2. (45.7 g, 73%, MS: [M+H]+ = 452.4)

### (Preparation Example 10-3) Preparation of Compound 10

Compound 10 was prepared by the same method as the preparation of compound 7, except that Intermediate 10-2 was used instead of Intermediate 7-2 and 2-([1,1'-biphenyl]-2-yl)-4-chloro-6-phenyl-1,3,5-triazine was used instead of 2-chloro-4,6-diphenyl-1,3,5-triazine. (21.4 g, 51%, MS: [M+H]+ = 634.8)

### Preparation Example 11: Preparation of Compound 11

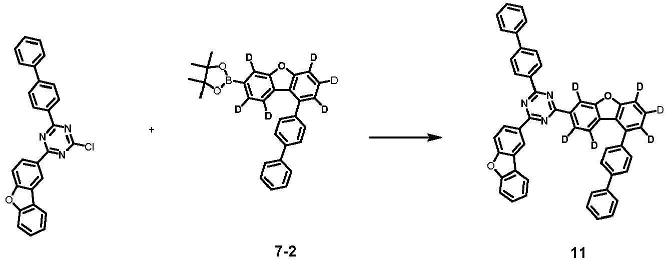

Compound 11 was prepared using the same method as for compound 7, except that 2-([1,1'-biphenyl]-4-yl)-4-chloro-6-(dibenzo[b,d]furan-2-yl)-1,3,5-triazine was used instead of 2-chloro-4,6-diphenyl-1,3,5-triazine. (33.5 g, 67%, MS: [M+H]+ = 724.9)

### Preparation Example 12: Preparation of Compound 12

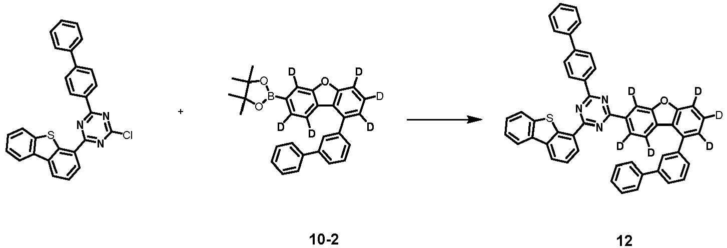

Compound 12 was prepared in the same manner as compound 10, except that 2-([1,1'-biphenyl]-4-yl)-4-chloro-6-(dibenzo[b,d]thiophen-4-yl)-1,3,5-triazine was used instead of 2-([1,1'-biphenyl]-2-yl)-4-chloro-6-phenyl-1,3,5-triazine. (31.1 g, 63%, MS:
[M+H]+ = 740.9)

### Preparation Example 13: Preparation of Compound 13

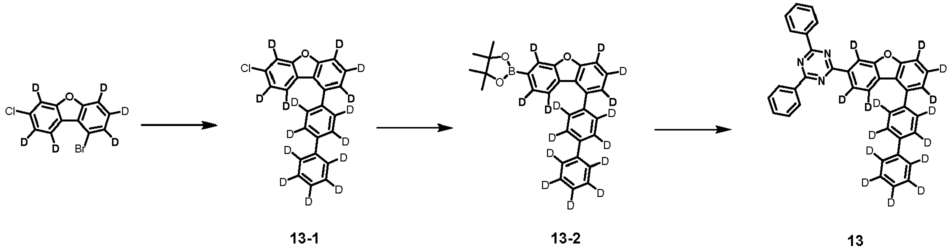

### (Preparation Example 13-1) Preparation of Intermediate 13-1

Intermediate 13-1 was prepared by the same method as the preparation of Intermediate 7-1, except that ([1,1'-biphenyl]-4-yl-d9)boronic acid was used instead of [1,1'-biphenyl]-4-ylboronic acid. (34.7 g, 54%, MS: [M+H]+ = 370.9)

### (Preparation Example 13-2) Preparation of Intermediate 13-2

Intermediate 13-2 was prepared by synthesizing using the same method as the preparation of Intermediate 7-2. (53 g, 85%, MS: [M+H]+ = 462.4)

### (Preparation Example 13-3) Preparation of Compound 13

Compound 13 was prepared by the same method as the preparation of compound 7, except that Intermediate 13-2 was used instead of Intermediate 7-2 and 2-chloro-4,6-diphenyl-1,3,5-triazine was used instead of 2-chloro-4,6-diphenyl-1,3,5-triazine. (24.5 g, 67%, MS: [M+H]+ = 567.7)

### Preparation Example 14: Preparation of Compound 14

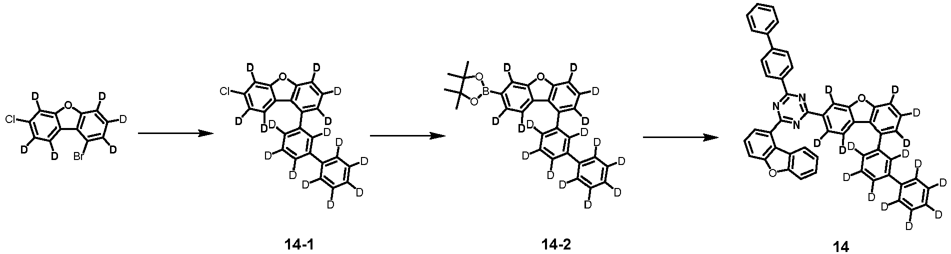

### (Preparation Example 14-1) Preparation of Intermediate 14-1

Intermediate 14-1 was prepared by the same method as the preparation of Intermediate 7-1, except that ([1,1'-biphenyl]-3-yl-d9)boronic acid was used instead of [1,1'-biphenyl]-4-ylboronic acid. (37.9 g, 59%, MS: [M+H]+ = 370.9)

### (Preparation Example 14-2) Preparation of Intermediate 14-2

Intermediate 14-2 was prepared by synthesizing using the same method as the preparation of Intermediate 7-2. (54.9 g, 88%, MS: [M+H]+ = 462.4)

### (Preparation Example 14-3) Preparation of Compound 14

Compound 14 was prepared by the same method as the preparation of compound 7, except that Intermediate 14-2 was used instead of Intermediate 7-2 and 2-chloro-4,6-diphenyl-1,3,5-triazine was used instead of 2-chloro-4,6-diphenyl-1,3,5-triazine. (32.3 g, 68%, MS: [M+H]+ = 733.9)

### Preparation Example 15: Preparation of Compound 15

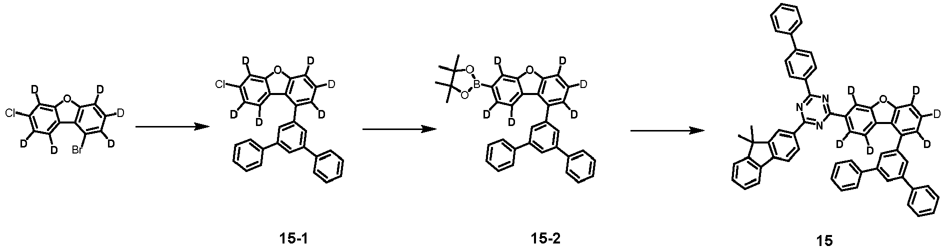

### (Preparation Example 15-1) Preparation of Intermediate 15-1

Intermediate 15-1 was prepared by the same method as the preparation of Intermediate 7-1, except that [1,1':3',1"-terphenyl]-5'-ylboronic acid was used instead of [1,1'-biphenyl]-4-ylboronic acid. (52.4 g, 69%, MS: [M+H]+ = 438)

### (Preparation Example 15-2) Preparation of Intermediate 15-2

Intermediate 15-2 was prepared by synthesis using the same method as the preparation of Intermediate 7-2. (52 g, 86%, MS: [M+H]+ = 529.5)

### (Preparation Example 15-3) Preparation of Compound 15

Compound 15 was prepared by the same method as the preparation of compound 7, except that Intermediate 15-2 was used instead of Intermediate 7-2 and 2-([1,1'-biphenyl]-4-yl)-4-chloro-6-(9,9-dimethyl-9H-fluoren-2-yl)-1,3,5-triazine was used instead 2-chloro-4,6-diphenyl-1,3,5-triazine. (29.1 g, 62%, MS: [M+H]+ = 827)

### Preparation Example 16: Preparation of Compound 16

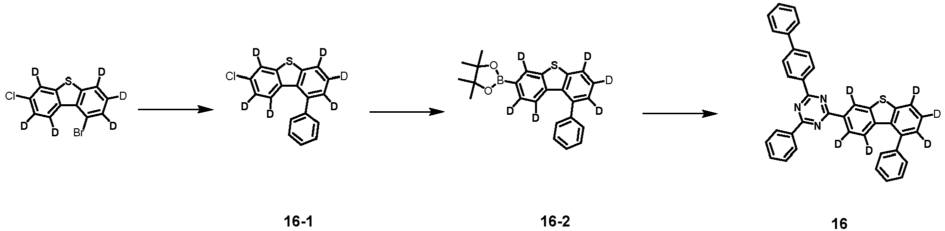

### (Preparation Example 16-1) Preparation of Intermediate 16-1

Intermediate 16-1 was prepared by the same method as the preparation of Intermediate 1-1, except that 1-bromo-7-chlorodibenzo[b,d]thiophene-2,3,4,6,8,9-d6, phenylboronic acid was used instead of 1-bromo-7-chlorodibenzo[b,d]furan-2,3,4,6,8,9-d6, [1,1'-biphenyl]-4-ylboronic acid. (31. 7 g, 64%, MS: [M+H]+ = 301.8)

### (Preparation Example 16-2) Preparation of Intermediate 16-2

Intermediate 16-2 was prepared by synthesizing using the same method as the preparation of Intermediate 1-2. (57.4 g, 88%, MS: [M+H]+ = 393.4)

### (Preparation Example 16-3) Preparation of Compound 16

Compound 16 was prepared by the same method as for the preparation of compound 1, except that Intermediate 16-2 was used instead of Intermediate 1-2, and 2-([1,1'-biphenyl]-4-yl)-4-chloro-6-phenyl-1,3,5-triazine was used instead of Intermediate 1-2, 2-([1,1'-biphenyl]-4-yl)-4-chloro-6-phenyl-1,3,5-triazine. (24.1 g, 55%, MS: [M+H]+ = 574.8)

### Preparation Example 17: Preparation of Compound 17

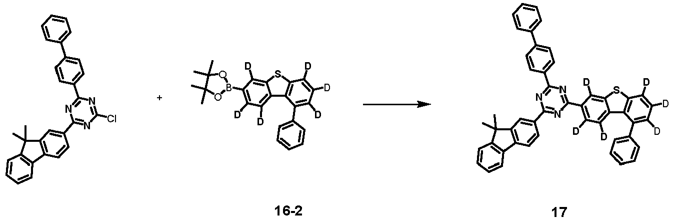

Compound 17 was prepared using the same method as for compound 16, except that 2-([1,1'-biphenyl]-4-yl)-4-chloro-6-(9,9-dimethyl-9H-fluoren-2-yl)-1,3,5-triazine was used instead of 2-([1,1'-biphenyl]-4-yl)-4-chloro-6-phenyl-1,3,5-triazine. (34.8 g, 66%, MS: [M+H]+ = 690.9)

### Preparation Example 18: Preparation of Compound 18

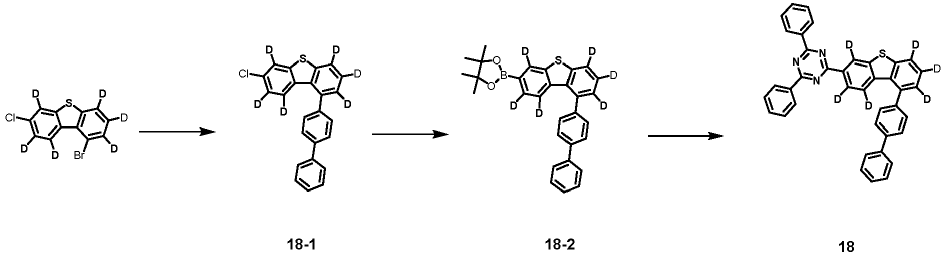

### (Preparation Example 18-1) Preparation of Intermediate 18-1

Intermediate 18-1 was prepared using the same method as the preparation of Intermediate 16-1, except that [1,1'-biphenyl]-4-ylboronic acid was used instead of phenylboronic acid. (44.1 g, 71%, MS: [M+H]+ = 377.9)

### (Preparation Example 18-2) Preparation of Intermediate 18-2

Intermediate 18-2 was prepared by synthesizing using the same method as that of Intermediate 16-2. (43.5 g, 70%, MS: [M+H]+ = 469.2)

### (Preparation Example 18-3) Preparation of Compound 18

Compound 18 was prepared by the same method as compound 16, except that Intermediate 18-2 was used instead of Intermediate 16-2 and 2-chloro-4,6-diphenyl-1,3,5-triazine was used instead of 2-([1,1'-biphenyl]-4-yl)-4-chloro-6-phenyl-1,3,5-triazine. (19.5 g, 53%, MS: [M+H]+ = 574.8)

### Preparation Example 19: Preparation of Compound 19

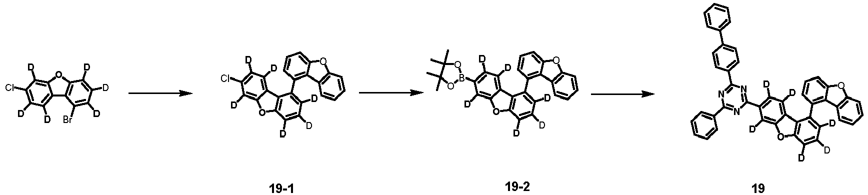

### (Preparation Example 19-1) Preparation of Intermediate 19-1

Intermediate 19-1 was prepared by the same method as the preparation of Intermediate 7-1, except that dibenzo[b,d]furan-1-ylboronic acid was used instead of [1,1'-biphenyl]-4-ylboronic acid. (34.5 g, 53%, MS: [M+H]+ = 375.9)

### (Preparation Example 19-2) Preparation of Intermediate 19-2

Intermediate 19-2 was prepared by synthesizing the same method as the preparation of Intermediate 7-2. (46 g, 74%, MS: [M+H]+ = 467.4)

### (Preparation Example 19-3) Preparation of Compound 19

Compound 19 was prepared by the same method as the preparation of compound 7, except that Intermediate 19-2 was used instead of Intermediate 7-2 and 2-([1,1'-biphenyl]-4-yl)-4-chloro-6-phenyl-1,3,5-triazine was used instead of 2-chloro-4,6-diphenyl-1,3,5-triazine. (27.9 g, 67%, MS: [M+H]+ = 648.8)

### Preparation Example 20: Preparation of Compound 20

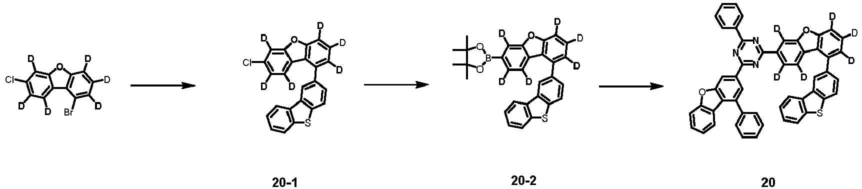

### (Preparation Example 20-1) Preparation of Intermediate 20-1

Intermediate 20-1 was prepared by the same method as the preparation of Intermediate 7-1, except that dibenzo[b,d]thiophen-2-ylboronic acid was used instead of [1,1'-biphenyl]-4-ylboronic acid. (36.7 g, 54%, MS: [M+H]+ = 391.9)

### (Preparation Example 20-2) Preparation of Intermediate 20-2

Intermediate 20-2 (51.5 g, 80%, MS: [M+H]+ = 483.4) was prepared by synthesizing the same method as the preparation of Intermediate 7-2.

### (Preparation Example 20-3) Preparation of Compound 20

Compound 20 (32.8 g, 70%, MS: [M+H]+ = 754.9) was prepared by the same method as the preparation of Compound 7, except that Intermediate 20-2 was used instead of Intermediate 7-2 and 2-chloro-4-phenyl-6-(1-phenyldibenzo[b,d]furan-3-yl)-1,3,5-triazine was used instead of 2-chloro-4,6-diphenyl-1,3,5-triazine.

### [Examples]

### Example 1

A glass substrate on which ITO (indium tin oxide) was coated as a thin film to a thickness of 100 nm was put into distilled water in which a detergent was dissolved, and ultrasonically cleaned. At this time, a product manufactured by Fischer Co. was used as the detergent, and distilled water filtered twice using a filter manufactured by Millipore Co. was used as the distilled water. After the ITO was cleaned for 30 minutes, ultrasonic cleaning was repeated twice using distilled water for 10 minutes. After the cleaning with distilled water was completed, the substrate was ultrasonically cleaned with solvents of isopropyl alcohol, acetone, and methanol, dried, and then transferred to a plasma cleaner. In addition, the substrate was cleaned for 5 minutes using oxygen plasma and then transferred to a vacuum depositor.

The following compound HI-A was thermally vacuum-deposited to a thickness of 60 nm on the prepared ITO transparent electrode to form a hole injection layer.

A first hole transport layer having a thickness of 5 nm was formed by vacuum-depositing the compound HAT on the hole injection layer, and a second hole transport layer having a thickness of 50 nm was formed by vacuum-depositing the compound HT-A on the first hole transport layer.

The following compound HT-B was thermally vacuum-deposited to a thickness of 45 nm on the hole transport layer to form an electron blocking layer. On the electron blocking layer, the compound 1 prepared above and the compound GD were vacuum-deposited at a weight ratio of 85:15 to a thickness of 40 nm to form a light emitting layer. On the light emitting layer, the following compound ET-A was vacuum-deposited to a thickness of 5 nm to form a hole blocking layer. On the hole blocking layer, the following compound ET-B and the following compound LiQ were vacuum-deposited at a weight ratio of 1:1 to form an electron injection and transport layer having a thickness of 35 nm.

After depositing lithium fluoride (LiF) to a thickness of 1 nm on the electron injection and transport layer, aluminum was deposited to a thickness of 100 nm to form a cathode, thereby manufacturing an organic light emitting device.

In the above process, the deposition rate of the organic material was maintained at 0.04 nm/sec to 0.09 nm/sec, the deposition rate of lithium fluoride was maintained at 0.03 nm/sec, and the deposition rate of aluminum was maintained at 0.2 nm/sec. In addition, the degree of vacuum during the deposition was maintained at 1×10⁻⁷ torr to 5×10⁻⁵ torr.

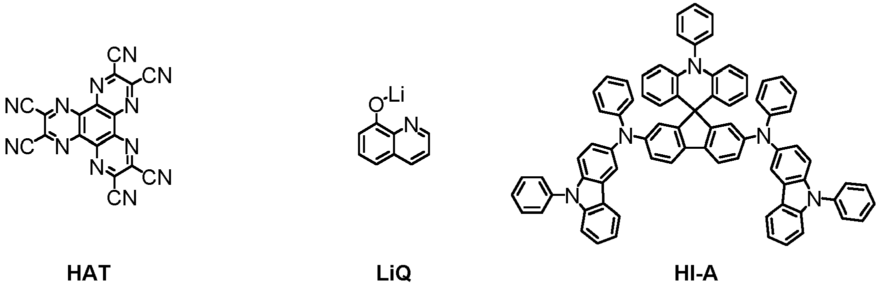

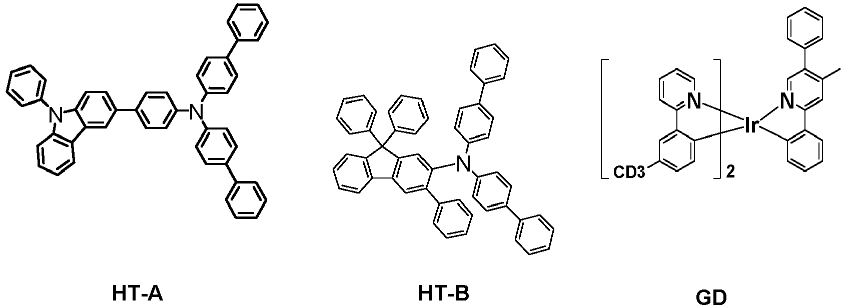

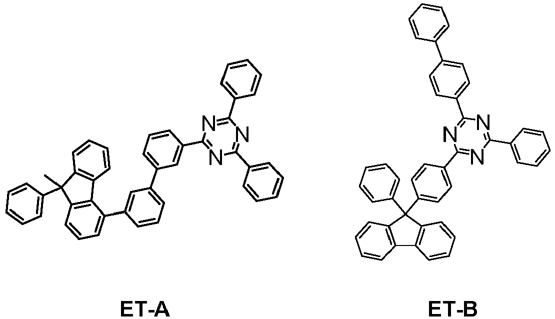

### Examples 2 to 25 and Comparative Examples 1 to 5

An organic light emitting device was manufactured in the same manner as in Example 1, except that the compound shown in Table 1 was used instead of the compound 1. For reference, in Examples 21 to 25 and Comparative Examples 4 to 5, organic light emitting devices were manufactured by using the compounds shown in Table 1 below at a weight ratio of 1:1 instead of the compound 1-1. In Example 21, for example, compound 1 and compound H-2 were used at a weight ratio of 1:1 instead of the compound 1 in Example 1. In Table 1 below, compounds H-2 and C1 to C3 are respectively as follows.

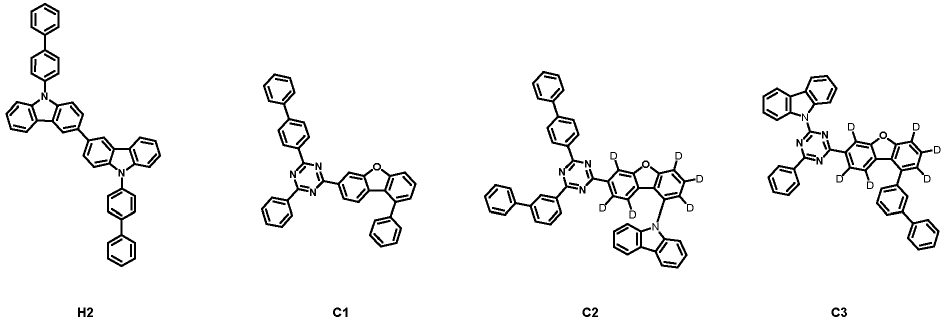

### Experimental Example

For the organic light emitting devices prepared in Examples and Comparative Examples, the voltage, efficiency, and emission color were measured by applying a current of 10 mA/cm², and the results are shown in Table 1. In addition, the time taken until the initial luminance (1600 nit) decreases to 95% when applying a current of 20 mA/cm² was measured in order to confirm the lifespan (T95, hr) of the organic light emitting devices.

**[Table 1]**

| Category | Compound in light emitting layer | Voltage (V) (@10mA/cm²) | Efficiency (cd/A) (@10mA/cm²) | Emission color | T₉₅ (hr) (@20mA/cm²) |
|---|---|---|---|---|---|
| Example 1 | Compound 1 | 3.07 | 67.4 | Green | 98 |
| Example 2 | Compound 2 | 3.06 | 68.3 | Green | 94 |
| Example 3 | Compound 3 | 3.03 | 67.7 | Green | 90 |
| Example 4 | Compound 4 | 3.04 | 67.5 | Green | 93 |
| Example 5 | Compound 5 | 3.09 | 67.5 | Green | 95 |
| Example 6 | Compound 6 | 3.05 | 66.6 | Green | 97 |
| Example 7 | Compound 7 | 3.02 | 66.2 | Green | 102 |
| Example 8 | Compound 8 | 3.04 | 67.7 | Green | 101 |
| Example 9 | Compound 9 | 3.09 | 68.6 | Green | 95 |
| Example 10 | Compound 10 | 3.05 | 67.2 | Green | 92 |
| Example 11 | Compound 11 | 3.01 | 67.4 | Green | 90 |
| Example 12 | Compound 12 | 3.06 | 66.9 | Green | 80 |
| Example 13 | Compound 13 | 3.03 | 67.5 | Green | 88 |
| Example 14 | Compound 14 | 3.08 | 67.4 | Green | 85 |
| Example 15 | Compound 15 | 3.06 | 68.7 | Green | 95 |
| Example 16 | Compound 16 | 3.04 | 66.3 | Green | 100 |
| Example 17 | Compound 17 | 3.01 | 66.4 | Green | 95 |
| Example 18 | Compound 18 | 3.02 | 68.5 | Green | 98 |
| Example 19 | Compound 19 | 3.09 | 67.9 | Green | 99 |
| Example 20 | Compound 20 | 3.08 | 68.4 | Green | 92 |
| Example 21 | Compound 1 Compound H-2 | 3.10 | 73.6 | Green | 95 |
| Example 22 | Compound 7, Compound H-2 | 3.09 | 75.5 | Green | 110 |
| Example 23 | Compound 11, Compound H-2 | 3.09 | 74.4 | Green | 100 |
| Example 24 | Compound 13, Compound H-2 | 3.06 | 71.3 | Green | 93 |
| Example 25 | Compound 19, Compound H-2 | 3.07 | 72.9 | Green | 102 |
| Comparative Example 1 | Compound C1 | 3.08 | 67.0 | Green | 50 |
| Comparative Example 2 | Compound C2 | 3.33 | 59.4 | Green | 65 |
| Comparative Example 3 | Compound C3 | 3.34 | 60.4 | Green | 62 |
| Comparative Example 4 | Compound C1, Compound H-2 | 3.11 | 73.4 | Green | 52 |
| Comparative Example 5 | Compound C3, Compound H-2 | 3.37 | 61.0 | Green | 65 |

In Table 1, Examples 1 to 20 and Comparative Examples 1 to 3 are examples of organic light emitting devices using a single host in the light emitting layer, and Examples 21 to 25 and Comparative Examples 4 to 5 are examples of devices using two types of hosts in the light emitting layer.

Referring to the Table 1, not only when a single host is used in the light emitting layer, but also when two types of hosts are used, the organic light emitting devices of Examples using the compound of Chemical Formula 1 of the present disclosure had higher efficiency, lower driving voltage and greatly improved lifespan than the organic light emitting devices of Comparative Examples.

From the above results, it can be confirmed that when the compound of Chemical Formula 1 is used as a host of an organic light emitting device, characteristics of low voltage, high efficiency, and long lifespan can be realized.

### [DESCRIPTION OF SYMBOLS]

| | | | |
|---|---|---|---|
| 1: | Substrate | 2: | Anode |
| 3: | Organic material layer | 4: | Cathode |
| 5: | Hole injection layer | 6: | Hole transport layer |
| 7: | Electron blocking layer | 8: | Light emitting layer |
| 9: | Hole blocking layer | 10: | Electron transport layer |
| 11: | Electron injection layer | 12: | Electron injection and transport layer |

## Claims

1. A compound represented by the following Chemical Formula 1: 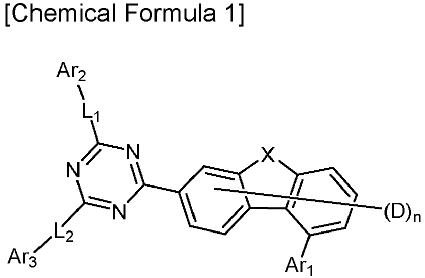 in the Chemical Formula 1,
n is an integer of 1 to 6,
X is O or S,
L₁ and L₂ are each independently a single bond; substituted or unsubstituted C₆₋₆₀ arylene; or substituted or unsubstituted C₂₋₆₀ heteroarylene containing at least one selected from the group consisting of O, S, Si, P and B,
Ar₁ is cyano; substituted or unsubstituted C₆₋₆₀ aryl; or substituted or unsubstituted C₂₋₆₀ heteroaryl containing at least one selected from the group consisting of O, S, Si, P and B, and
Ar₂ and Ar₃ are each independently substituted or unsubstituted C₆₋₆₀ aryl; or substituted or unsubstituted C₂₋₆₀ heteroaryl containing at least one selected from the group consisting of O, S, Si, P and B.

2. The compound of Claim 1,
wherein n is an integer of 4 to 6.

3. The compound of Claim 1,
wherein L₁ and L₂ are each independently a single bond; phenylene unsubstituted or substituted with at least one deuterium; or biphenylylene unsubstituted or substituted with at least one deuterium.

4. The compound of Claim 1,
wherein Ar₁ is cyano; phenyl; biphenylyl; terphenylyl; triphenylenyl; benzofuranyl; benzothiophenyl; dibenzofuranyl; or dibenzothiophenyl; and
the Ar₁ is unsubstituted or substituted with at least one deuterium.

5. The compound of Claim 1,
wherein Ar₂ and Ar₃ are each independently phenyl; phenyl substituted with at least one substituent selected from the group consisting of halogen, cyano, trimethylsilyl, C₁₋₄ alkyl, and C₁₋₄ alkenyl; biphenylyl; terphenylyl; naphthyl; 9,9-dimethylfluorenyl; 9,9-diphenylfluorenyl; triphenylenyl; chrysenyl; dibenzofuranyl; phenyldibenzofuranyl; dibenzothiophenyl; or phenyldibenzothiophenyl, and
Ar₂ and Ar₃ are each independently unsubstituted or substituted with at least one deuterium.

6. The compound of Claim 1,
wherein the compound represented by the Chemical Formula 1 is any one selected from the group consisting of:

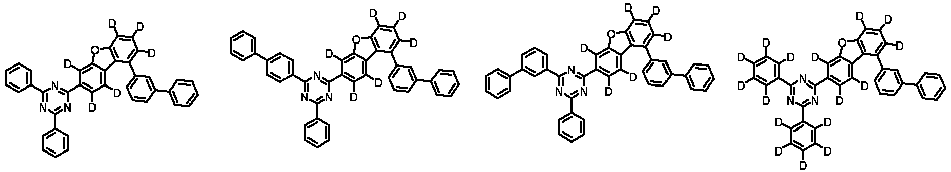

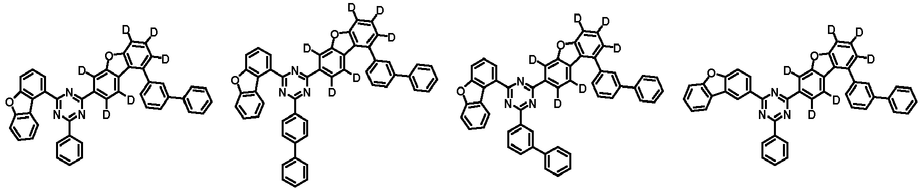

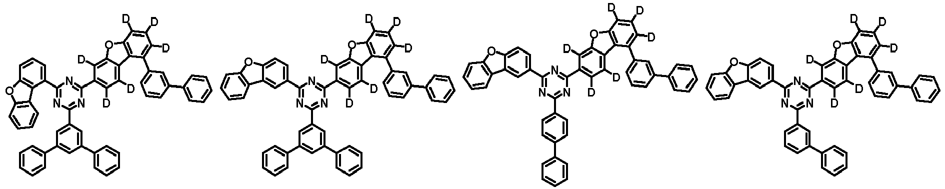

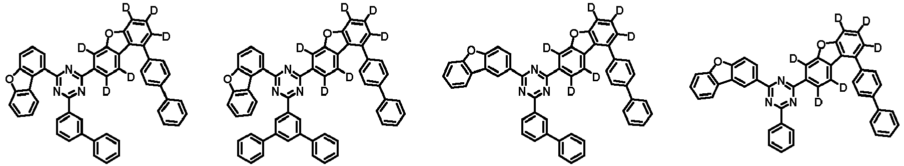

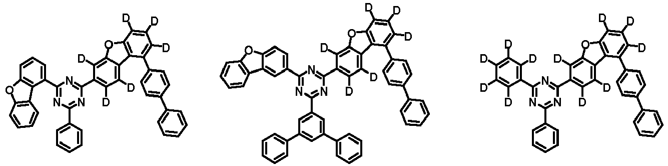

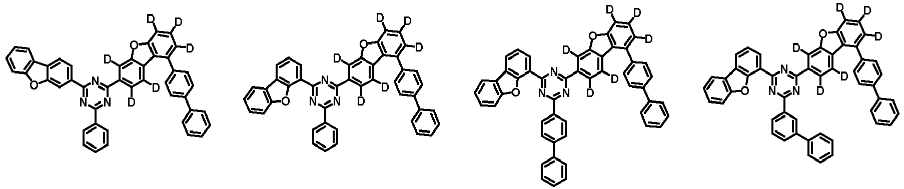

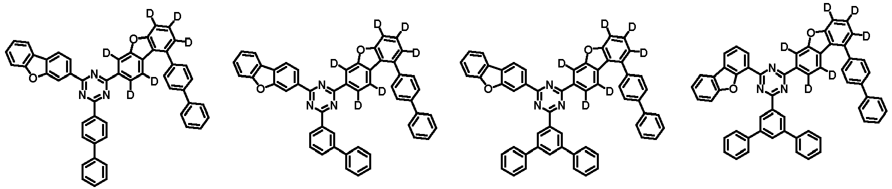

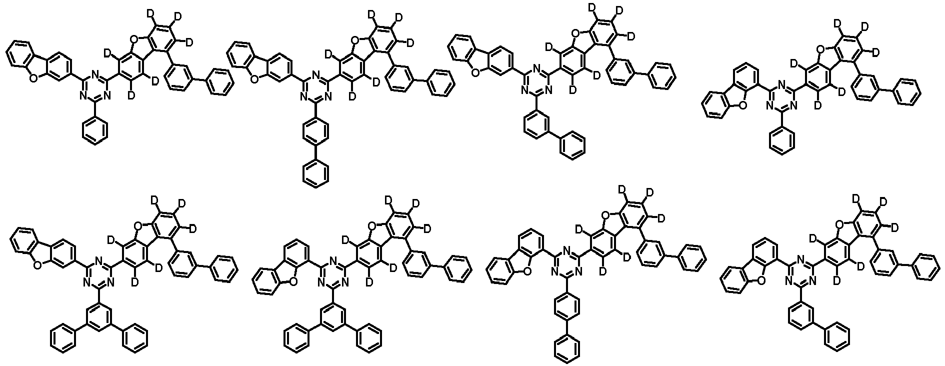

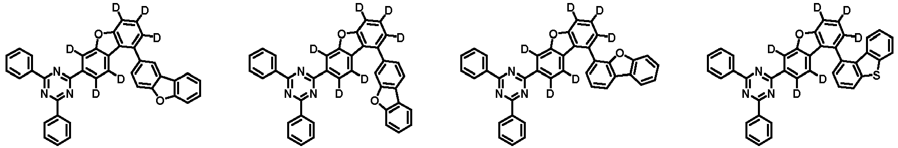

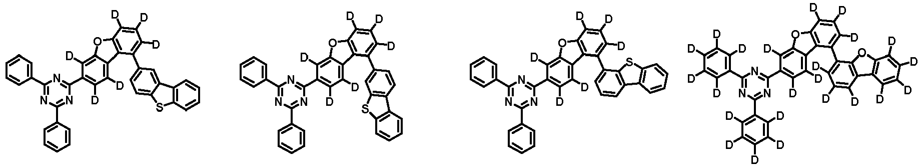

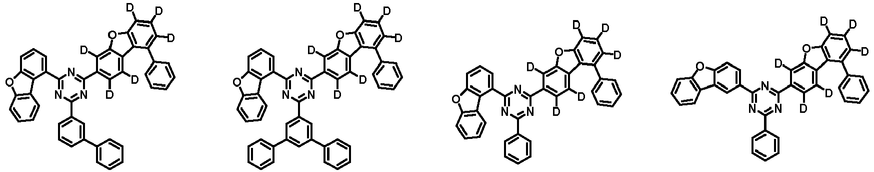

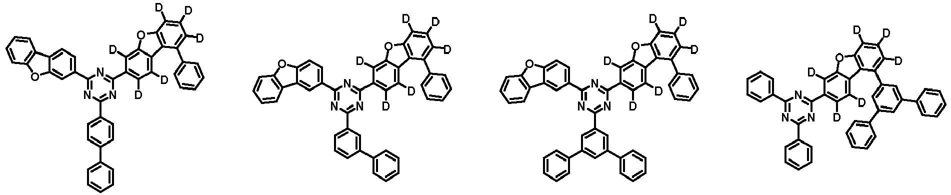

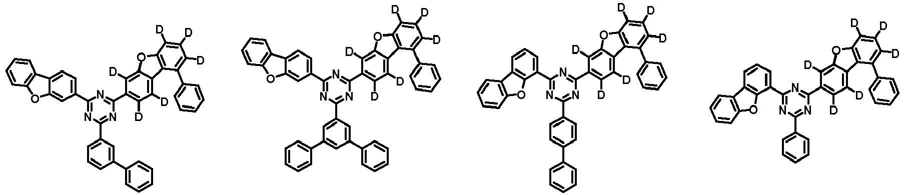

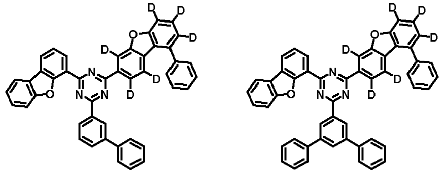

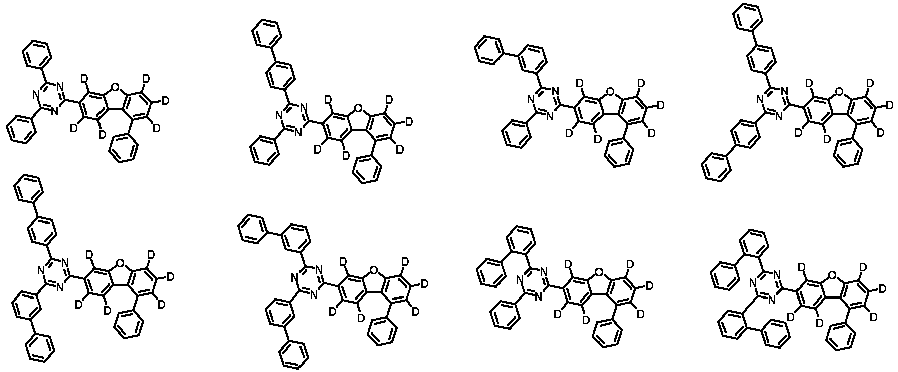

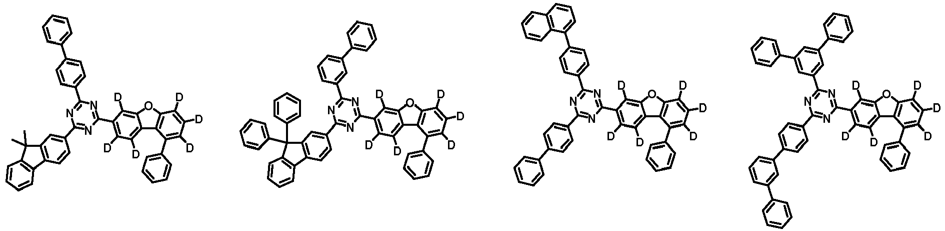

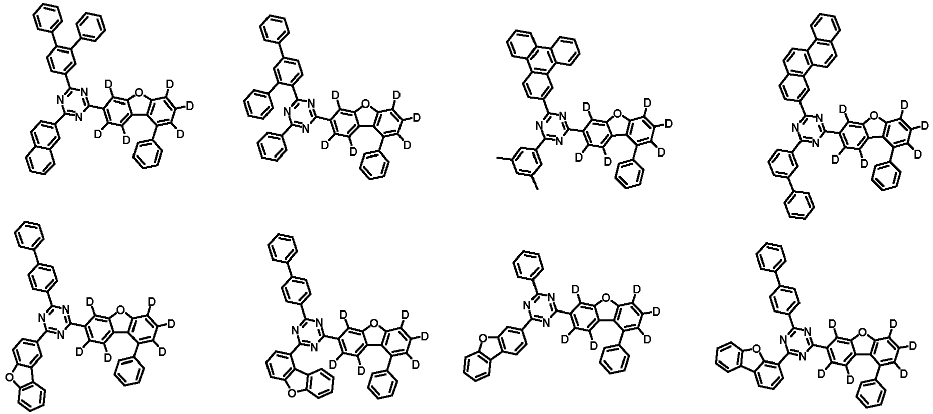

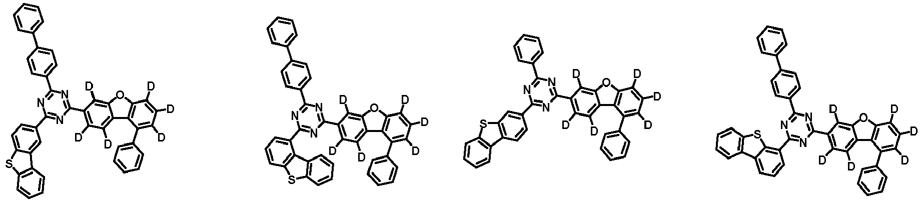

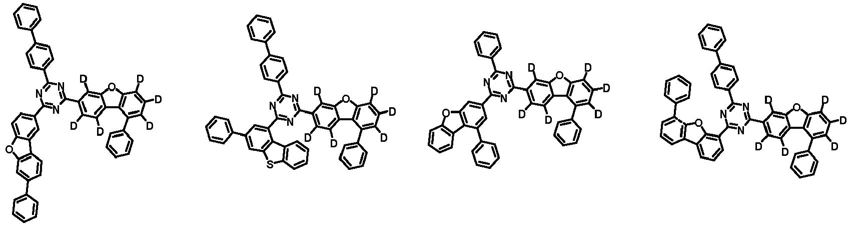

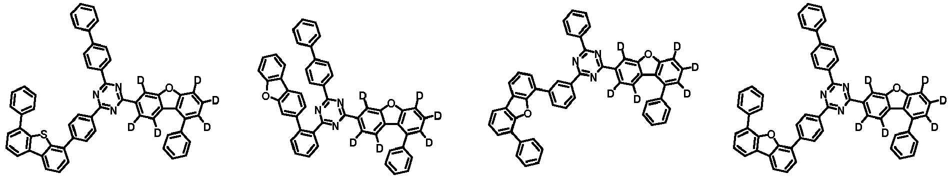

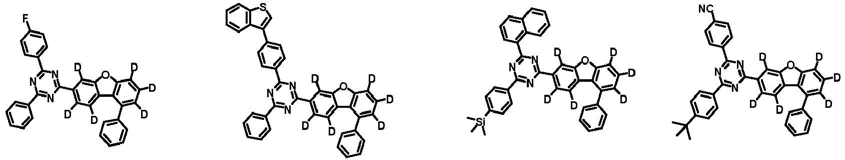

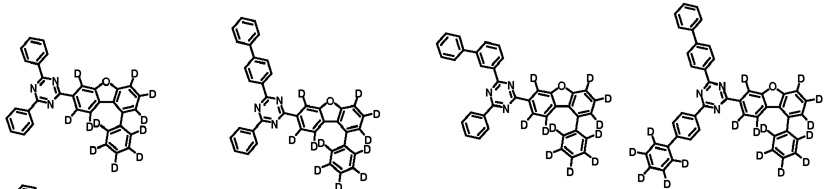

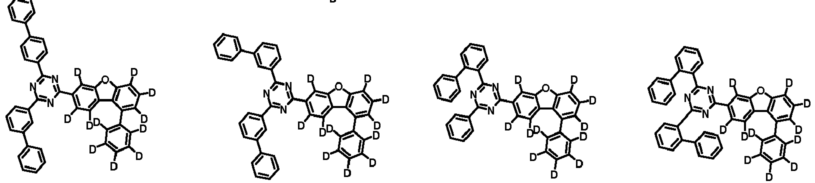

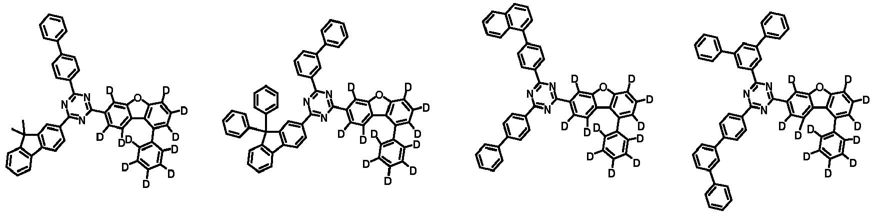

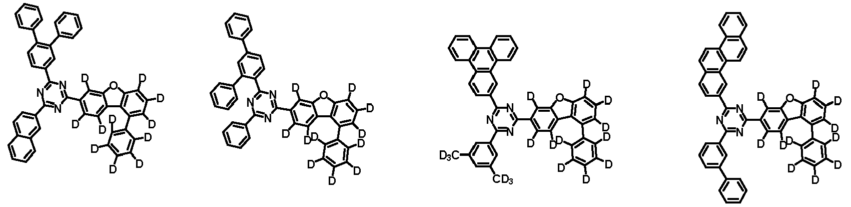

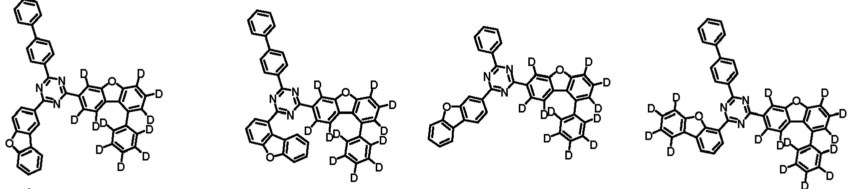

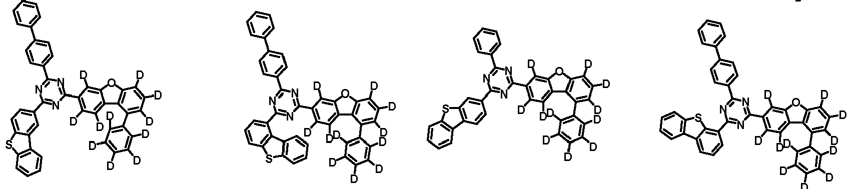

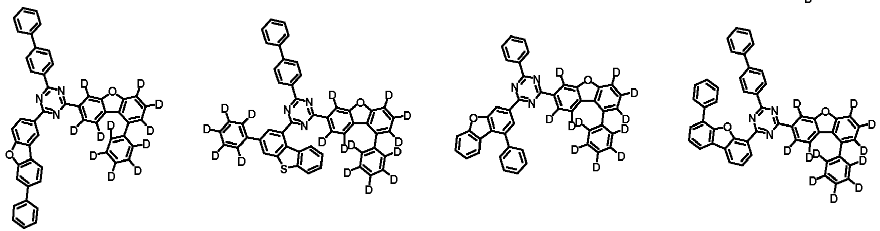

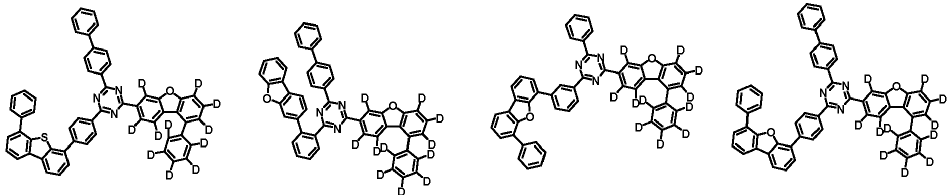

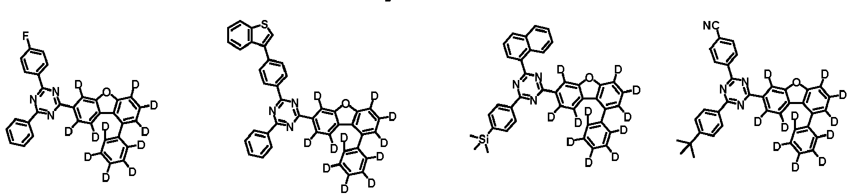

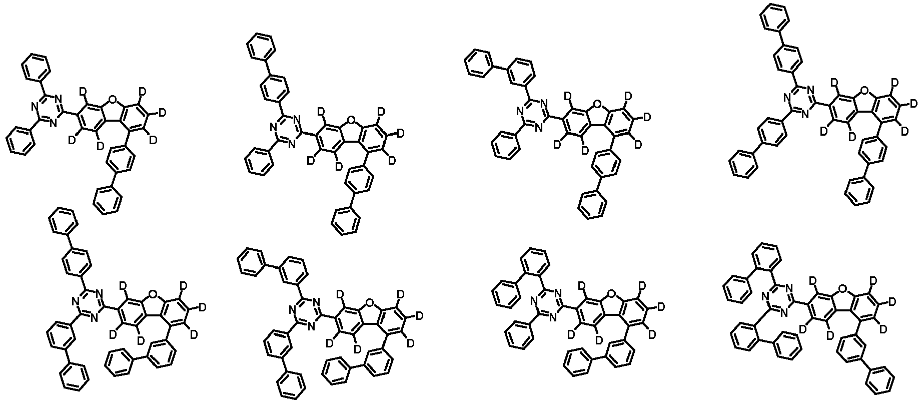

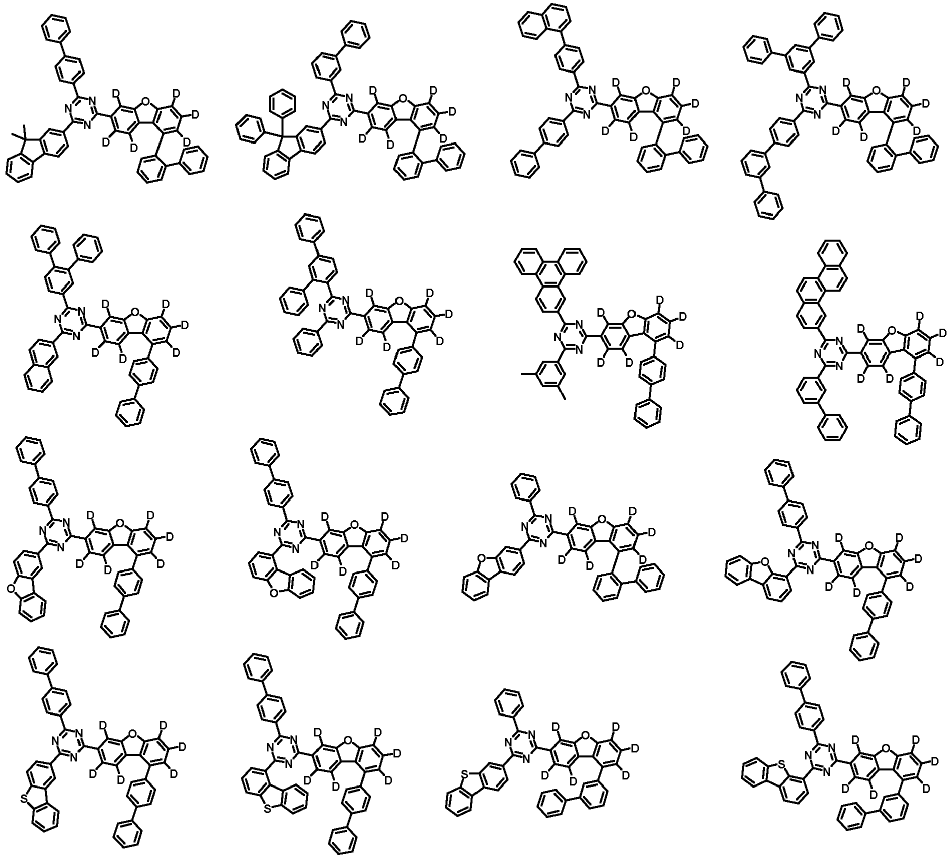

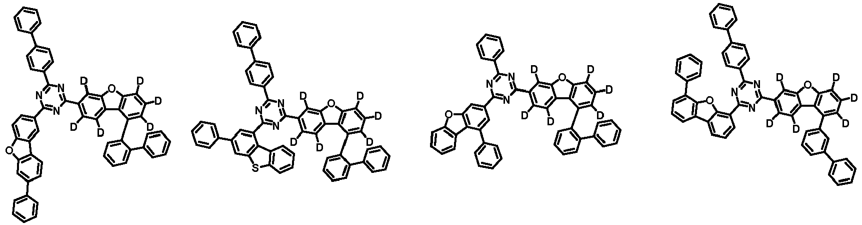

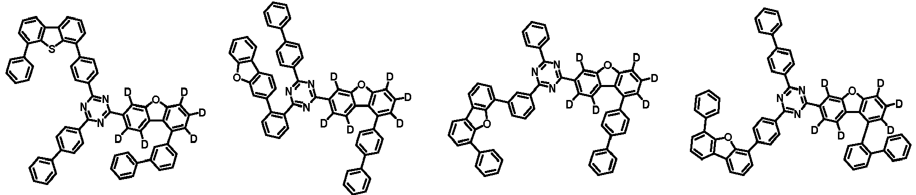

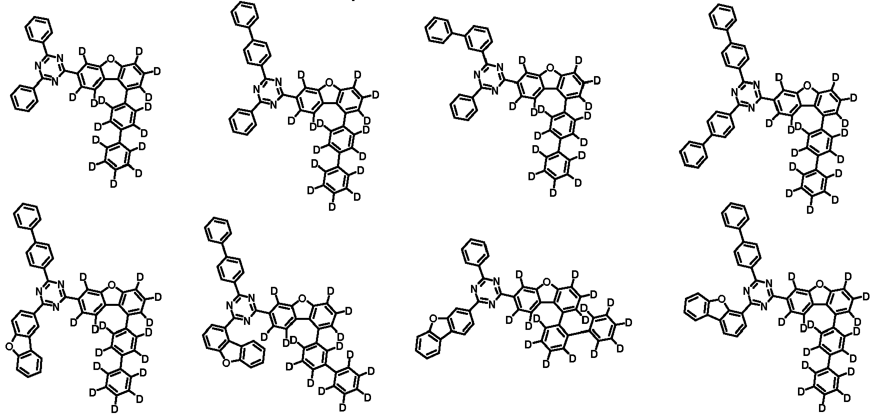

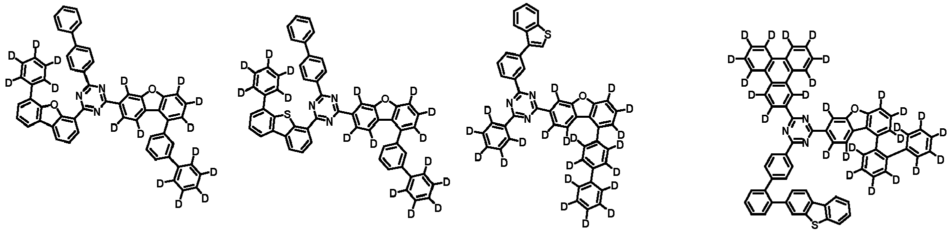

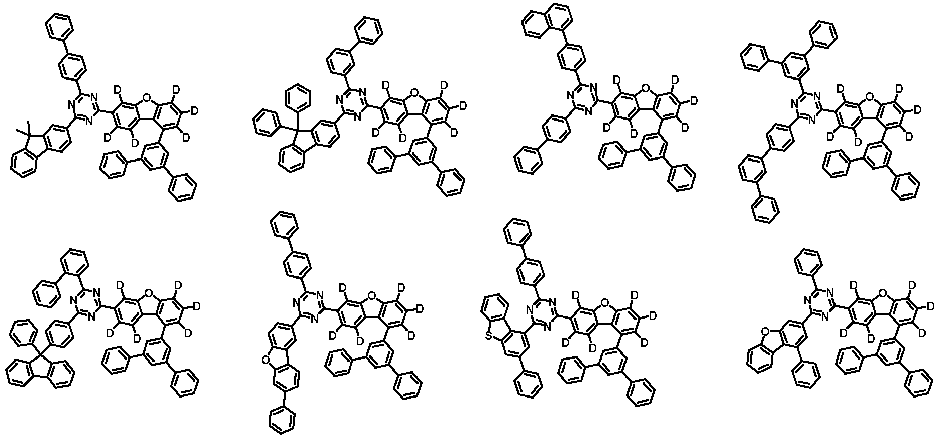

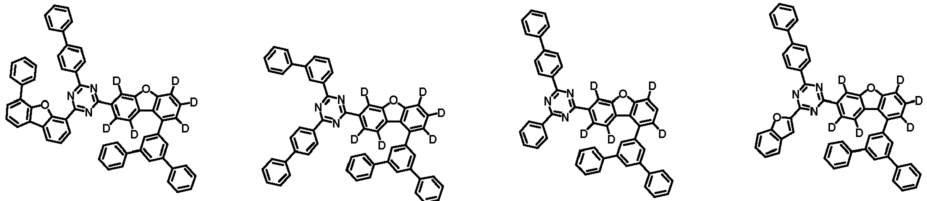

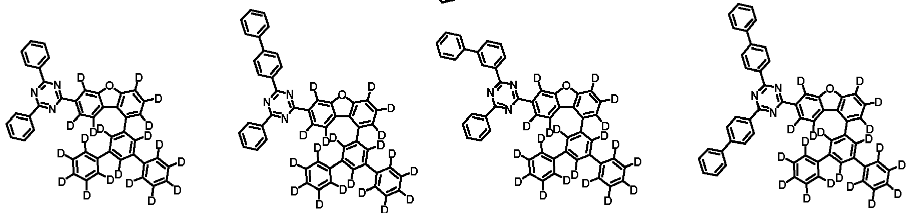

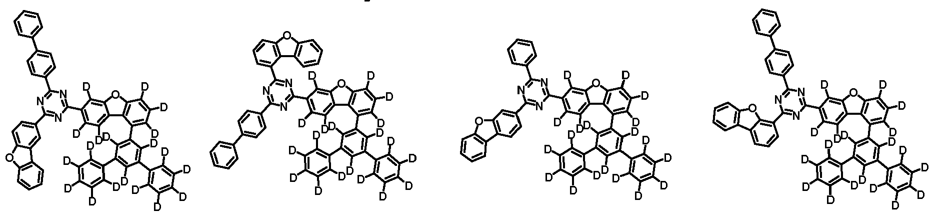

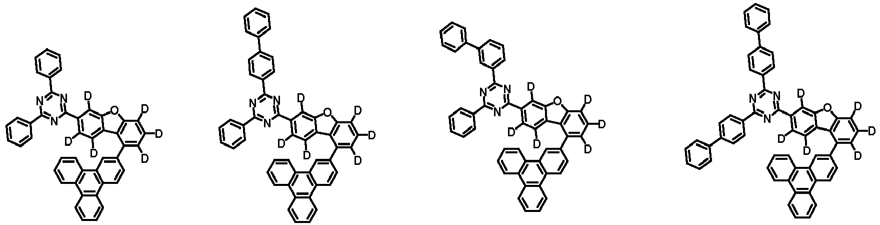

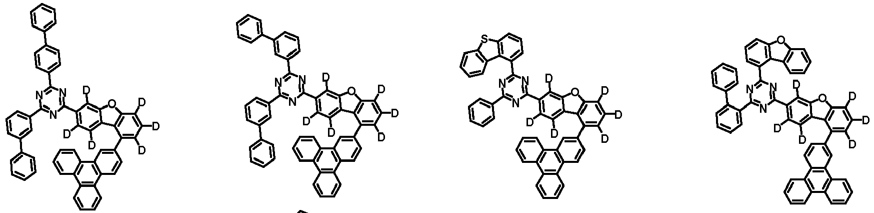

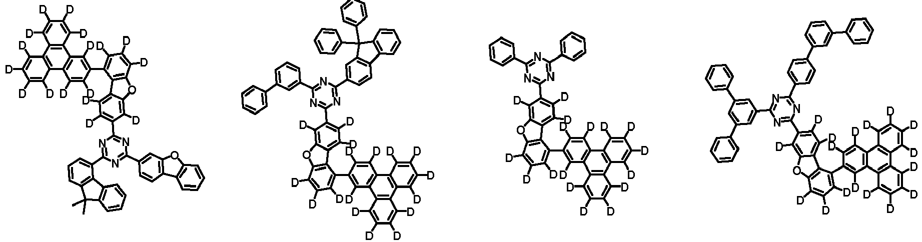

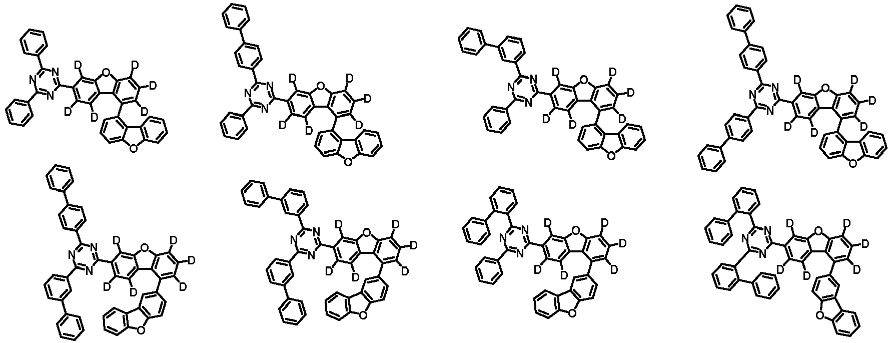

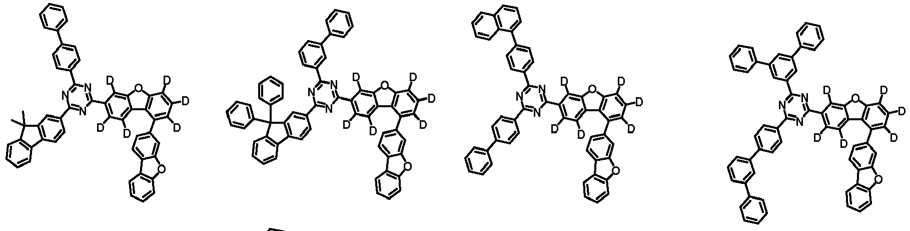

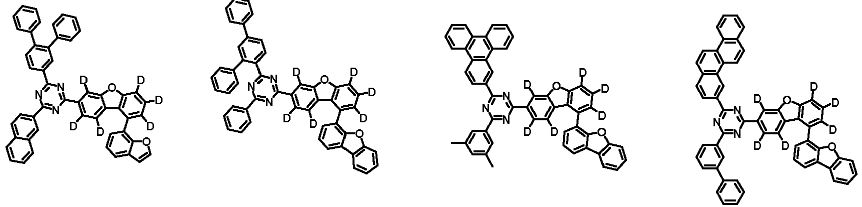

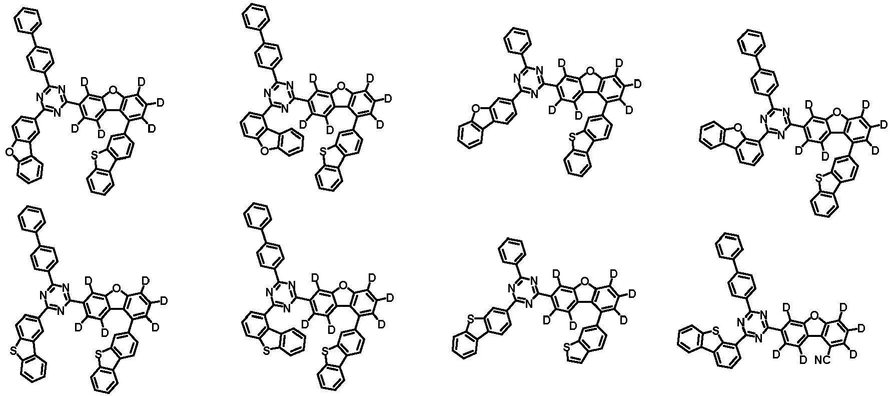

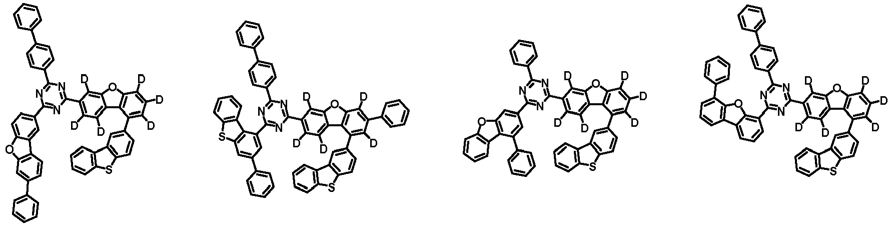

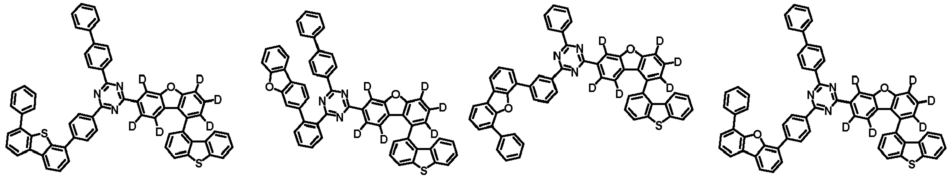

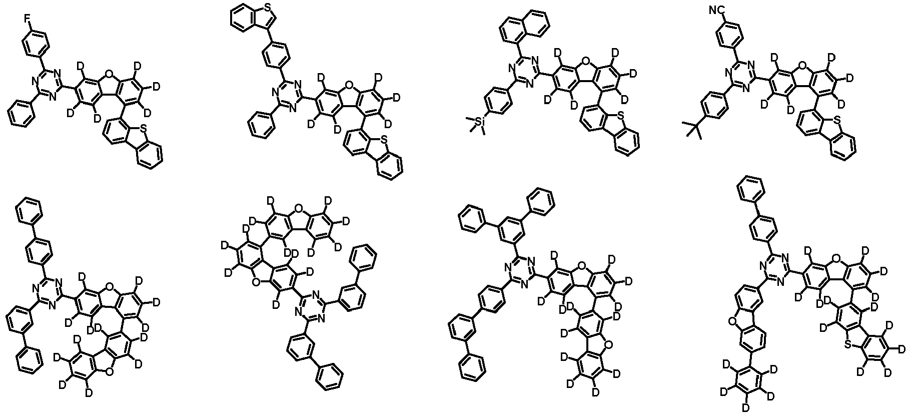

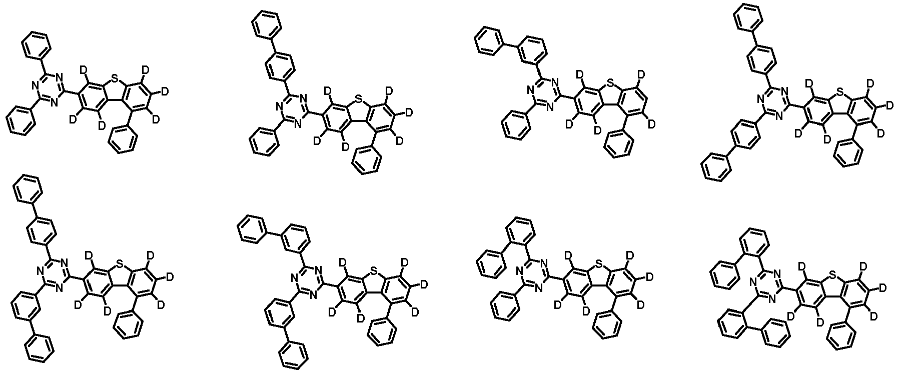

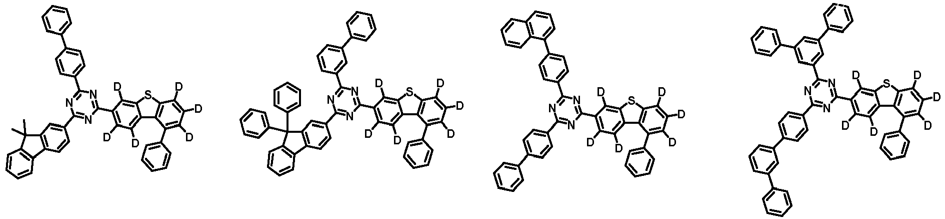

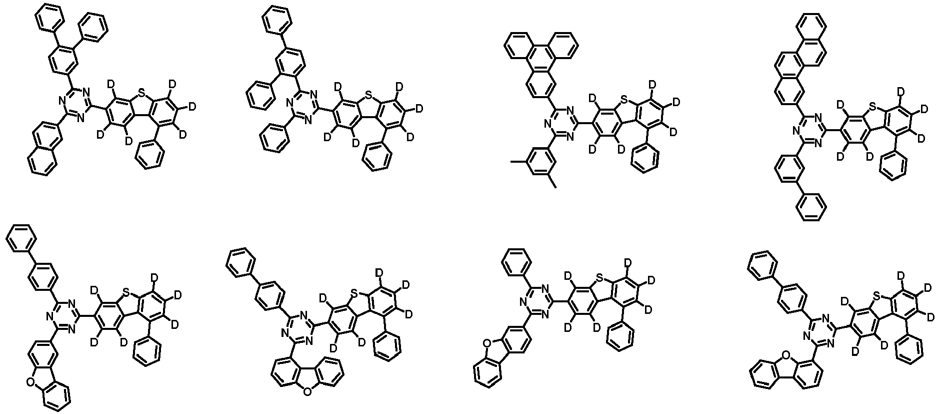

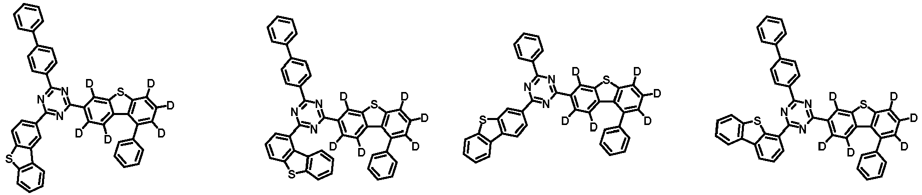

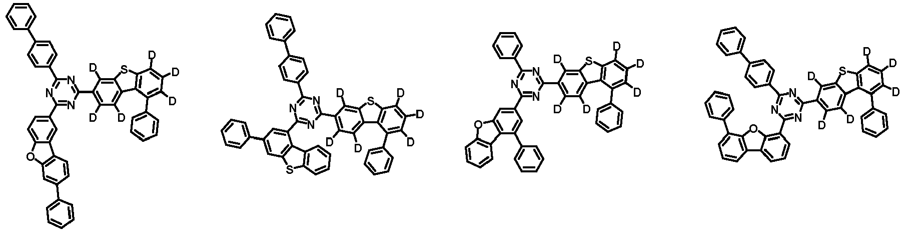

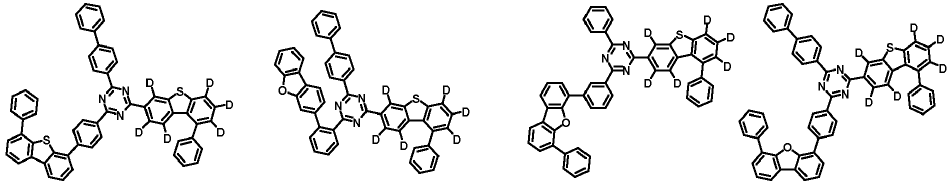

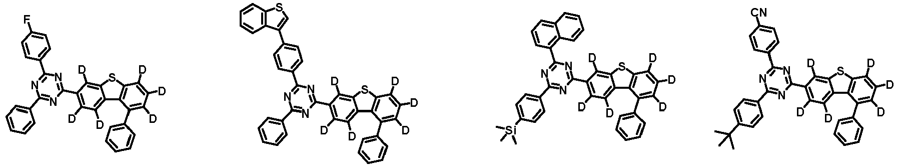

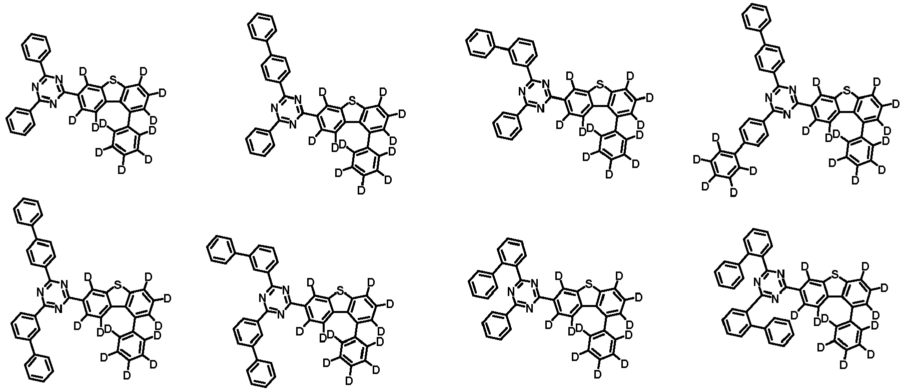

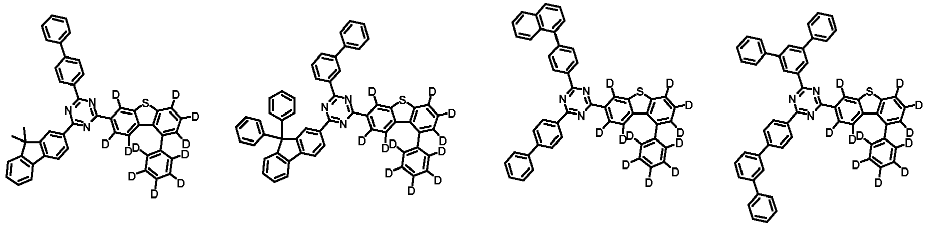

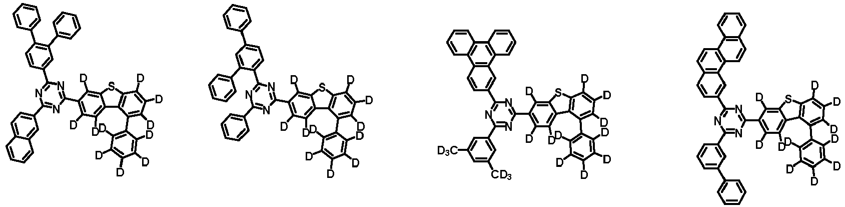

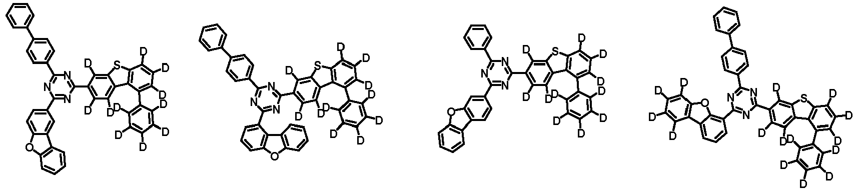

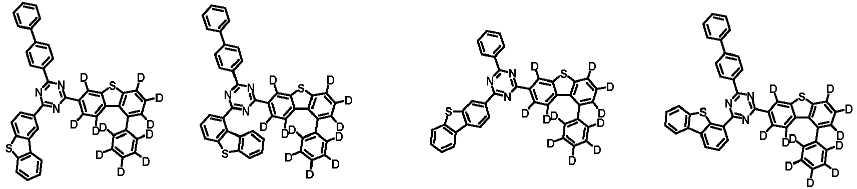

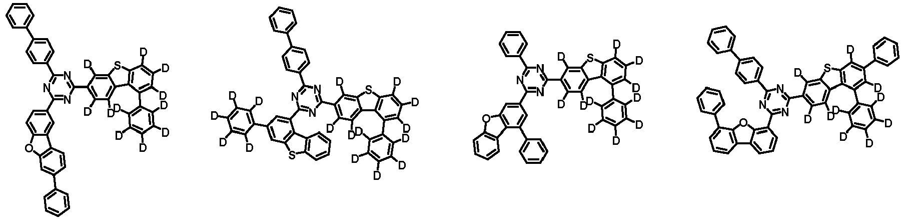

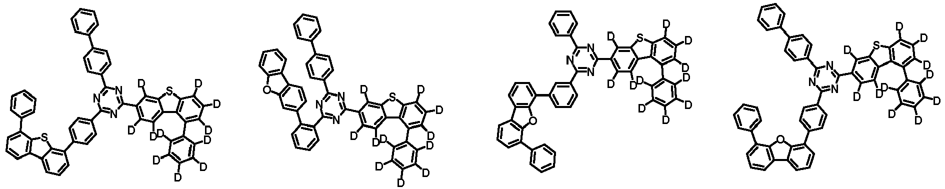

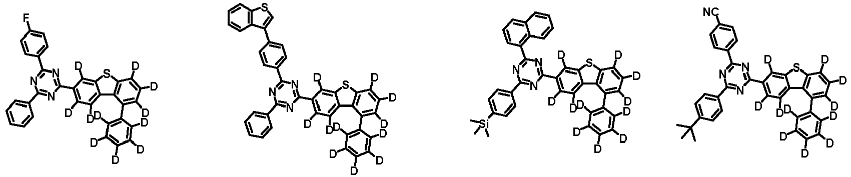

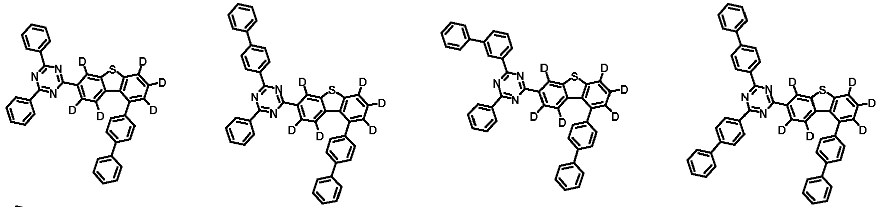

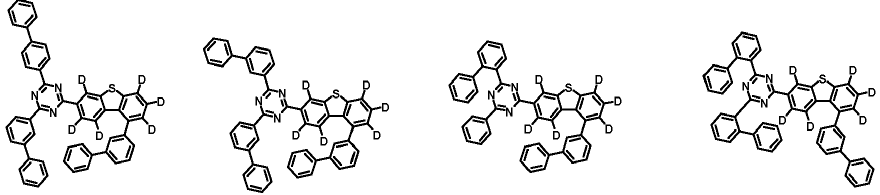

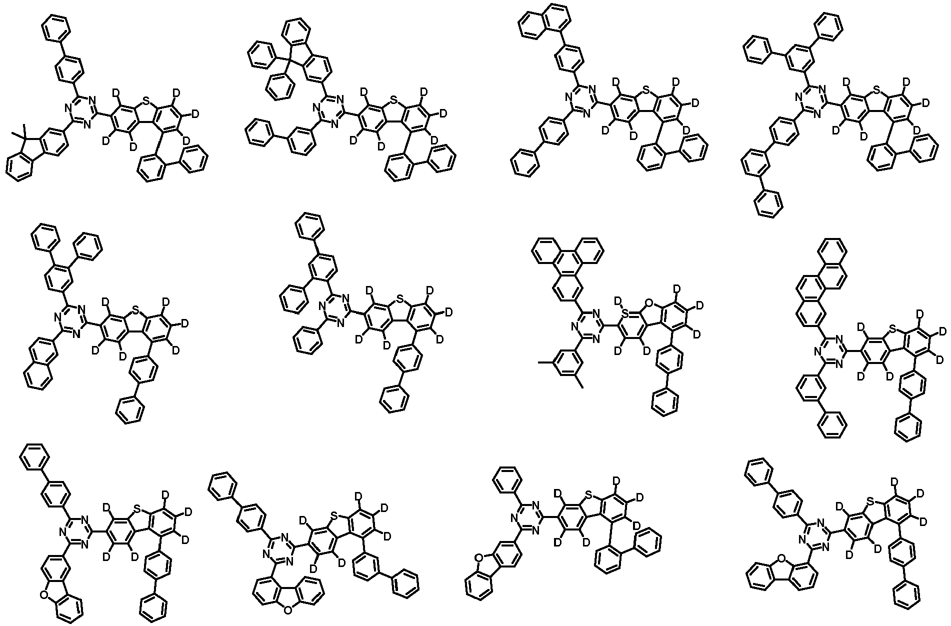

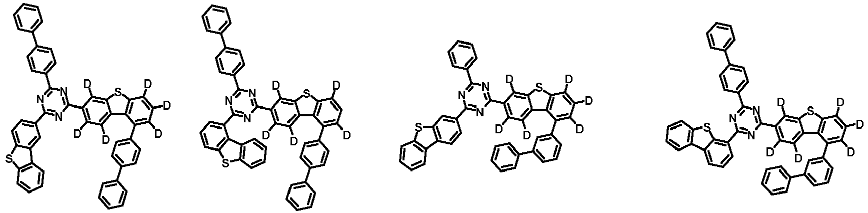

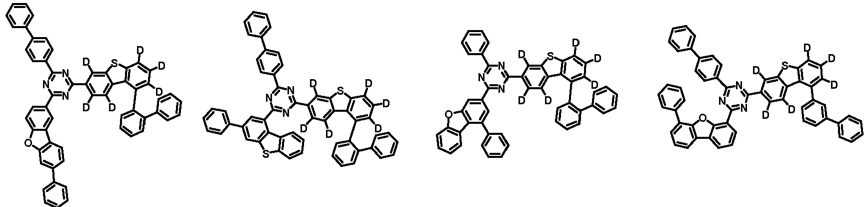

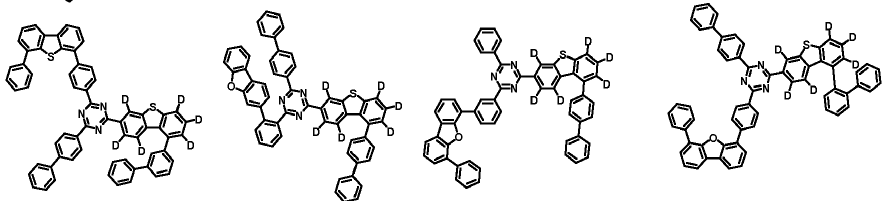

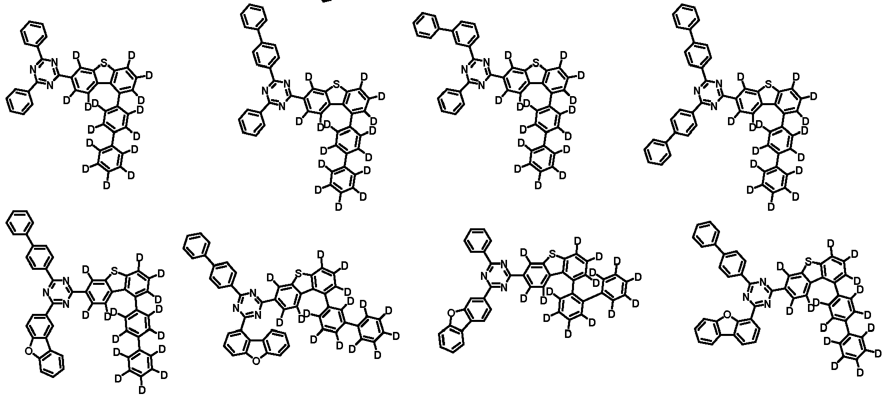

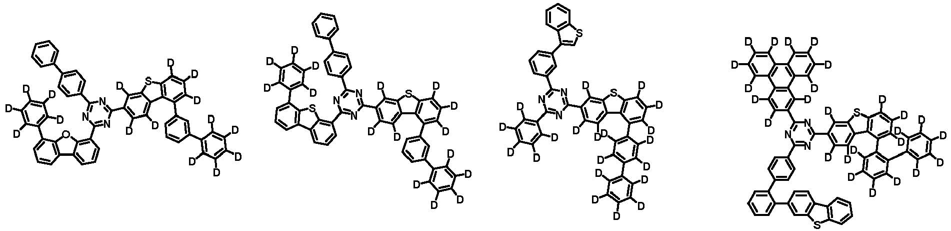

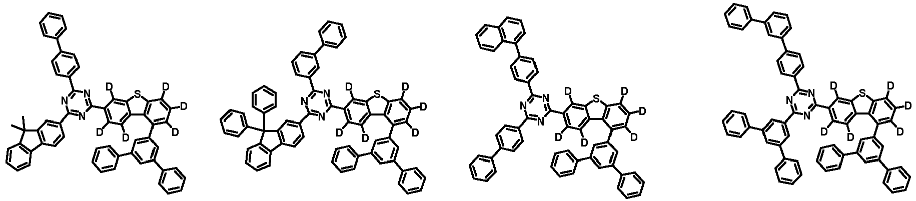

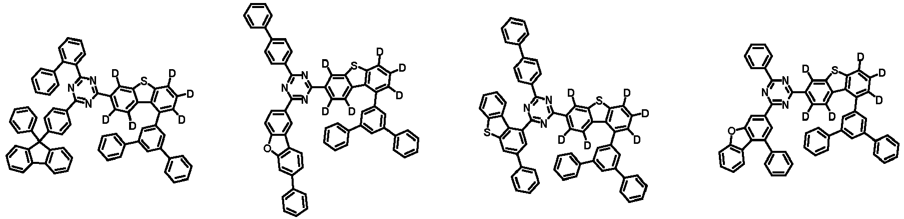

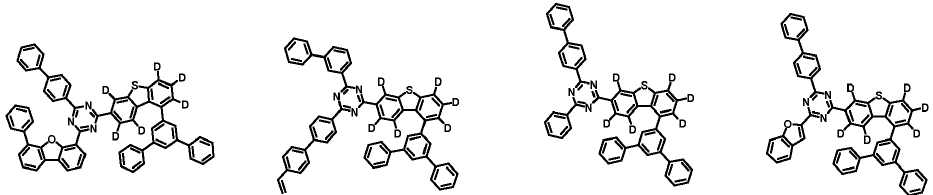

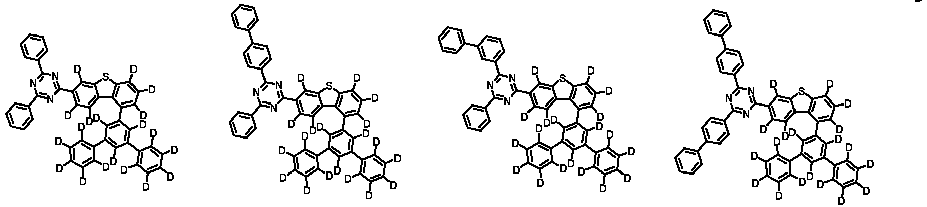

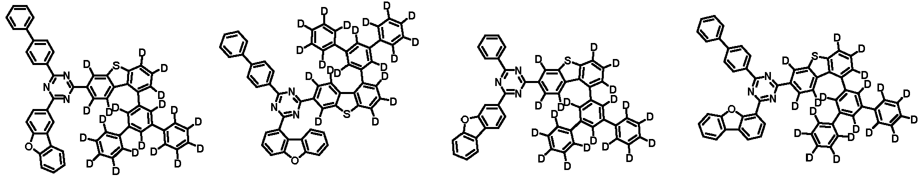

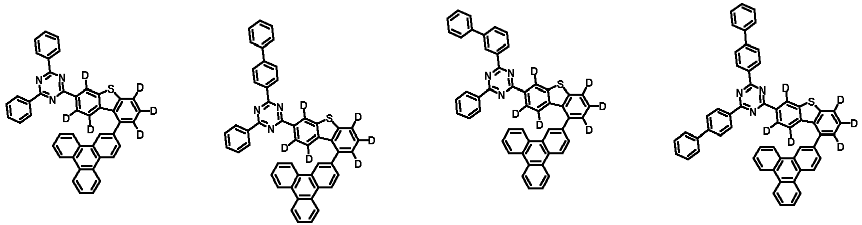

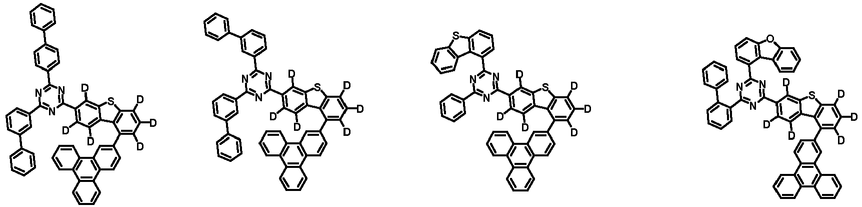

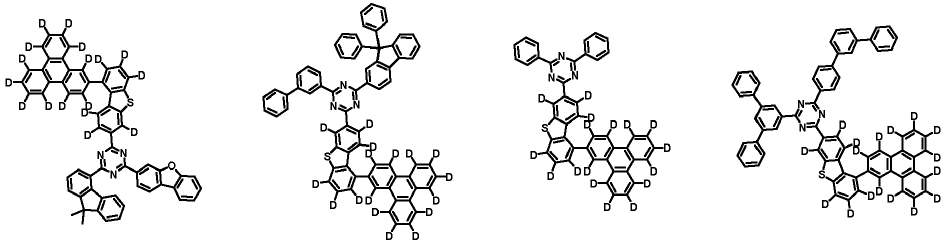

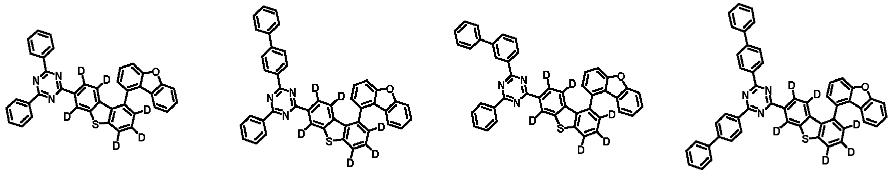

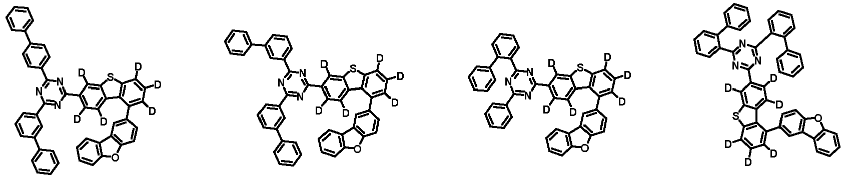

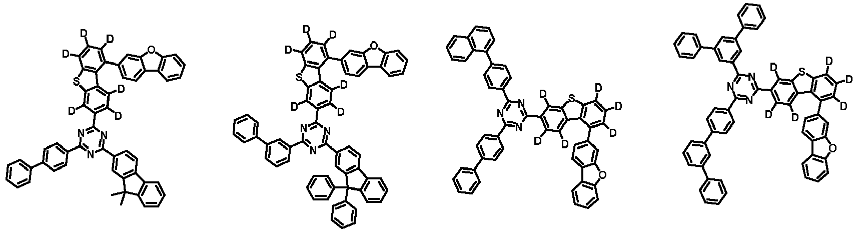

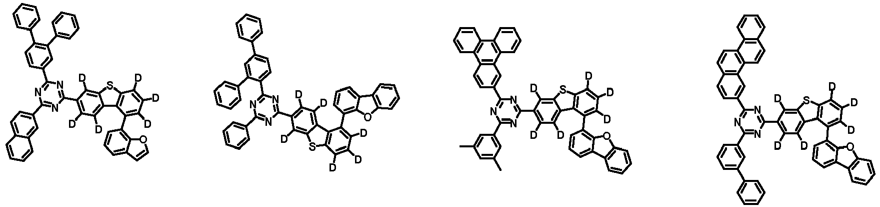

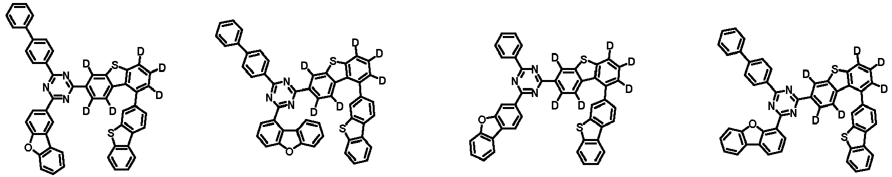

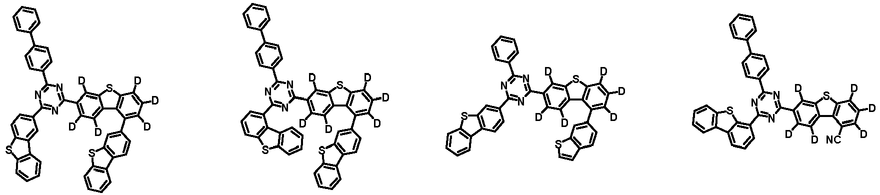

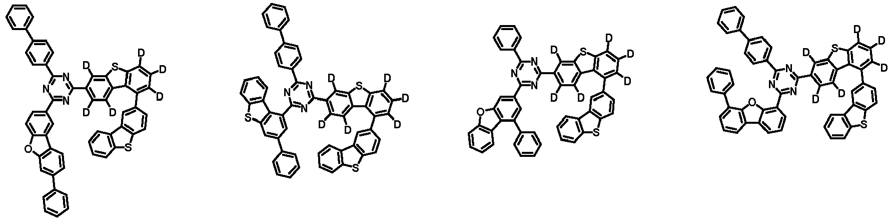

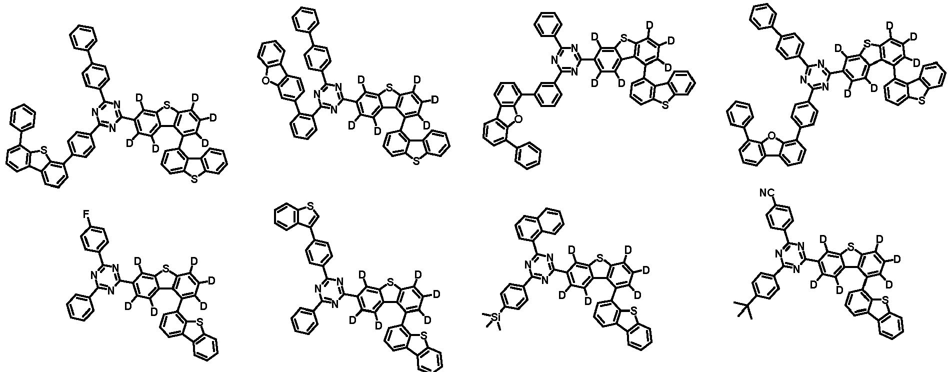

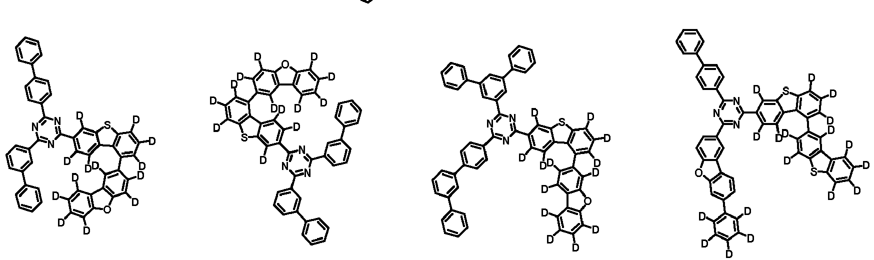

7. An organic light emitting device comprising: a first electrode; a second electrode that is provided opposite to the first electrode; and one or more organic material layers that are provided between the first electrode and the second electrode, wherein at least one layer of the organic material layers comprises the compound according to any one of Claims 1 to 6.

8. The organic light emitting device of Claim 7,
wherein the organic material layer comprising the compound is a light emitting layer.
